# EUROPEAN PATENT APPLICATION

(11) **EP 4 302 836 A1**
(43) Date of publication of application: **10.01.2024**
(21) Application number: 22182860.1
(22) Date of filing: 04.07.2022
(51) Int. Cl.: A61P 39/02, C07K 16/40

(54) **ANTIBODIES AGAINST SNAKE TOXINS CAUSING MUSCLE AND/OR TISSUE DAMAGE**

(71) Applicant: Danmarks Tekniske Universitet, 2800 Kongens Lyngby (DK); Universidad De Costa Rica, San José 11501 (CR)
(72) Inventor: LAUSTSEN-KIEL, Andreas Hougaard, 2800 Kongens Lyngby (DK); Sørensen, Christoffer Vinther, 2800 Kongens Lyngby (DK); LOMONTE, Bruno, San José (CR); Fernández, Julián, San José (CR)
(74) Representative: Høiberg P/S

(57) **Abstract**

In the field of recombinant antivenoms, a major hurdle to the development of antigen-binding proteins enabling sufficient toxin neutralization is the identification of antibodies with both high affinity and neutralizing activity against specific venom toxins, in particular toxins showing immune system evasion. Traditional antibody development approaches have not enabled to isolate antigen-binding proteins against venom toxins with such properties. The present disclosure describes the development of high-affinity human antibodies that show neutralizing effects against B. *asper* myotoxin II *in vitro* and *in vivo.*

The invention relates to antibodies capable of neutralizing B. *asper* myotoxin II. The disclosure also provides pharmaceutical compositions, kit of parts, and kits for the treatment and diagnosis of snake envenomation.

## Description

### Technical field

The present invention relates to antibodies capable of neutralizing snake toxins causing muscle and/or tissue damage, such as viper Myotoxin II.

### Background

Snakebite envenoming is a devastating neglected tropical disease that kills and permanently disables hundreds of thousands of people each year. The mainstay therapies against snakebite envenomings are antivenoms based on polyclonal antibodies, and while these have saved countless lives during the last 125 years, they have several unfavorable drawbacks, including a propensity to elicit adverse reactions, high costs, and a low content of therapeutically active antibodies.

A novel type of snakebite therapeutics, based on human monoclonal antibodies, is under development. Such human monoclonal antibodies can support the development of recombinant antivenoms with improved specificity, which combined with the human origin of the antibodies, can prove safer, more efficacious, and cost-competitive compared to current treatments.

Nevertheless, it is still a challenge to identify recombinant antibodies and produce recombinant antivenoms against snake toxins which show high affinity and therapeutic efficacy. This is also to be put in perspective with the extreme diversity of snake venoms, globally, which are typically complex mixtures of numerous toxins.

Among the areas particularly severely affected by snakebite envenoming are Central America and northern South America, where several snake species producing venom toxins causing muscle and tissue damage, *e.g*. the myotoxin II of the venomous pit viper, *Bothrops asper,* cause a large number of bites that result in mortality and morbidity. To date, no recombinant monoclonal antibody neutralizing the activity of myotoxin II of *Bothrops asper* has been discovered.

### Summary

The invention is defined in the attached claims.

Hundreds of thousands of people are killed or permanently disabled each year due to snakebite envenoming. The traditional therapies against these neglected tropical diseases are antivenoms derived from the plasma of hyperimmunized animals. While effective, these treatments are also associated with several drawbacks, such as the risk of causing adverse reactions, high costs, and low concentrations of therapeutically active antibodies. In addition, for some key toxins, such as PLA₂S and three-finger toxins, the toxins tend to be elusive to the immune system and therefore often fail to induce strong antibody levels in the production animal used for immunization.

To avoid these drawbacks, a novel type of snakebite therapeutics, based on human monoclonal antibodies, is under development. Using different platforms, such as the phage display technology, such human monoclonal antibodies can be selected specifically to target medically important snake venom toxins, for example of the Elapidæ family (Laustsen et al., 2015), providing a path towards the development of recombinant antivenoms with a very high therapeutic content of human origin.

However, an important challenge is the identification of novel antibodies which combine safety with high affinity, neutralizing activity and therapeutic efficacy against key snake toxins.

In particular, Central America and northern South America are areas still severely affected by snakebite envenoming, notably by the venomous pit viper, *Bothrops asper,* causing a large number of bites that results in mortality and morbidity or tissue necrosis and gangrene. The venom of B. *asper* contains a significant amount of potent myotoxic phospholipases A₂ (PLA₂s) and PLA₂-like proteins, one of which is myotoxin II (Lomonte et al., 1989). Myotoxin II and other myotoxic PLA₂s are responsible for serious clinical effects, such as tissue loss, disability, and amputation (Mora-Obando et al., 2014).

Despite being considered a key toxin to be neutralized by an antivenom for treatment to be efficacious in patients having fallen victim to envenoming by *B. asper,* no monoclonal antibody showing *B. asper* myotoxin II neutralizing activity could be identified so far.

The present invention however provides high affinity human recombinant monoclonal IgGs identified using phage display technology and a naïve human scFv-based antibody library, showing neutralizing activity against myotoxin II from *B*. *asper.*

The inventors demonstrate that the antibodies of the invention could neutralize the myotoxic effects caused by myotoxin II from *B*. *asper in vitro.*

Further, one antibody (TPL0039_05_B12) was able to fully neutralize the toxic effects in an *in vivo* rodent model at an unprecedented low toxin:antibody molar ratio of 1:1.

Finally, by co-administering one of these monoclonal IgGs (TPL0039_05_B12) with the polyclonal equine antivenom currently used against *B*. *asper* envenomings (ICP), the inventors show that these two treatments retain their neutralizing capabilities when administered together in an *in vivo* model. Combined, the results presented herein indicate that the human monoclonal antibodies disclosed herein are useful against *B*. *asper* envenoming either as an auxiliary treatment or as fortification agents for existing plasma-derived antivenoms.

Co-administration of monoclonal human antibodies of the invention is expected to lead to a decrease in the amount of total therapeutic product required for neutralization of snake toxins. In theory, a lower total antibody amount is plausible since monoclonal antibodies would neutralize the toxins that the polyclonal antivenoms poorly neutralize. Thereby the polyclonal antivenoms would only be required to neutralize their favored toxins, which should require less antivenom compared to the amount needed to also neutralize the difficult-to-neutralize targets.

A first aspect of the invention relates to an antibody capable of neutralizing myotoxin II of SEQ. ID. NO: 1 or a toxin sharing at least 60%, preferably at least 70%, yet more preferably at least 80%, such as at least 90%, for example at least 95%, such as at least 98% sequence identity therewith.

A second aspect of the invention relates to an antibody comprising or consisting of a heavy chain variable (VH) region comprising a heavy chain complementarity-determining region 3 (CDR-H3) comprising or consisting of SEQ. ID. NO: 4 , or a variant thereof, wherein in said variant up to 3 amino acids have been altered, for example wherein 2 or 1 amino acid(s) have been altered.

A third aspect of the invention relates to an antibody comprising or consisting of:
a) a heavy chain variable (VH) region comprising:
   i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
   ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
   iii. a heavy chain complementarity-determining region 3 (CDR-H3) comprising or consisting of SEQ. ID. NO: 4 or a variant thereof, wherein in said variant up to 3 amino acids have been altered, for example wherein 2 or 1 amino acid(s) have been altered;
   and
b) a light chain variable (VL) region comprising:
   i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
   ii. a light chain complementarity-determining region 2 (CDR-L2) comprising or consisting of SEQ. ID. NO: 6 or a variant thereof, wherein in said variant up to 3 amino acids have been altered, for example wherein 2 or 1 amino acid(s) have been altered; and
   iii. a light chain complementarity-determining region 3 (CDR-L3) comprising or consisting of SEQ. ID. NO: 7 or a variant thereof, wherein in said variant up to 3 amino acids have been altered, for example wherein 2 or 1 amino acid(s) have been altered.

A fourth aspect of the present invention relates to a pharmaceutical composition comprising an antibody as defined herein, and a pharmaceutically acceptable diluent, carrier and/or excipient.

A fifth aspect of the present invention relates to a kit-of-parts comprising
a) an antibody as described herein; and
b) an active agent suitable for the treatment of snake envenomation.

A sixth aspect of the present invention relates to an antibody as described herein, a pharmaceutical composition as described herein or a kit-of-parts as described herein for use in a method of treatment of snake envenomation in a subject.

A seventh aspect of the present invention relates to a method of treating snake envenomation which comprises administering to a subject in need thereof an effective amount of the antibody as described herein, the pharmaceutical composition as described herein or the kit-of-parts as described herein.

An eighth aspect of the present invention relate to the use of the antibody as described herein, the composition as described herein or the kit-of-parts as described herein for the manufacture of a medicament for the treatment of snake envenomation.

A ninth aspect of the present invention relates to a kit for detection and/or quantification of snake venom in a sample comprising:
- one or more antibodies, such as two antibodies, wherein at least one of said antibodies as described herein; and
- means for detection of a complex comprising said one or more antibodies bound to snake venom.

A tenth aspect of the present invention relates to an antibody as described herein for use in the diagnosis of snake envenomation in a subject.

An eleventh aspect of the present invention relates to the use of an antibody as described herein in an in vitro method for detection and/or diagnosis of snake envenomation in a subject.

A twelfth aspect of the present invention relates to a method for detecting snake venom in a sample, said method comprising:
- providing a sample for analysis;
- contacting the sample with one or more antibodies as defined herein to said sample; and
- detecting a complex comprising said one or more antibodies bound to a snake antigen.

### Description of Drawings

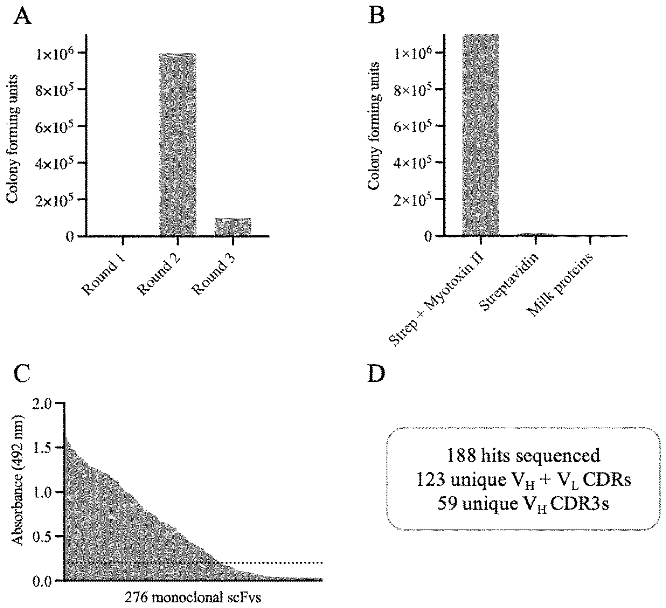

***Fig. 1******. Overview of results from phage display.*** A: Phage display panning results reported as colony forming units (CFUs) for each of the outputs from three rounds of panning. B: Assessment of the specificity of third round polyclonal phage output reported as CFU counts when panned against either myotoxin II of *Bothrops asper* or controls (streptavidin and milk proteins). C: Monoclonal scFv ELISA signals against myotoxin II of *Bothrops asper.* D: Summary of DNA sequencing results.

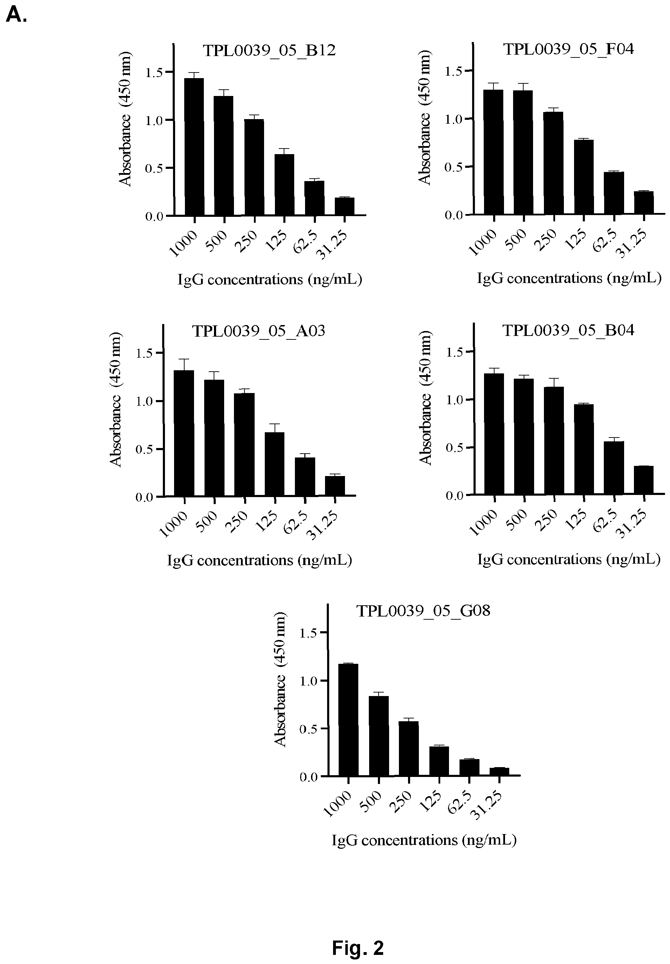

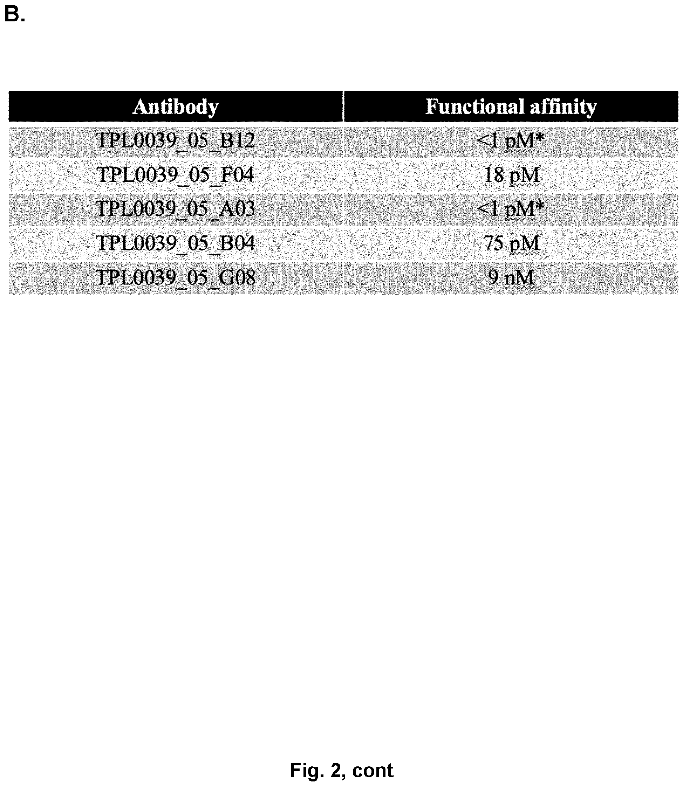

***Fig. 2******. IgG binding assessment.*** A: ELISAs showing binding to myotoxin II of *Bothrops asper* by each of the converted IgGs (IgG indicated at the top of each figure) tested in a dilution series. B: Functional affinity (avidity) of the five IgGs. * <1 pM means that the IgG was not observed to dissociate from myotoxin II of *Bothrops asper,* thereby obtaining the minimum value possible.

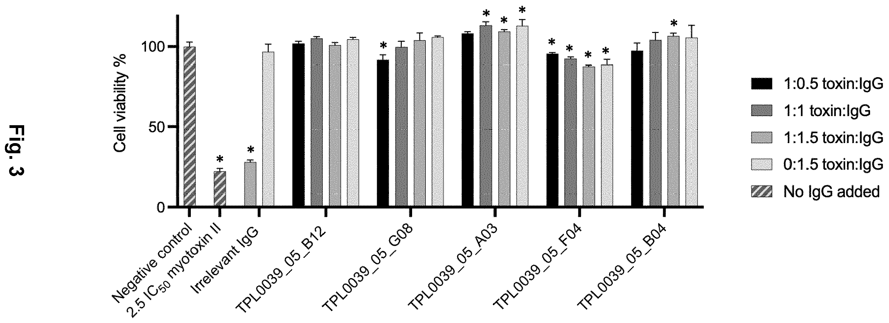

***Fig. 3******. Toxin:IgG ratios for full Bothrops asper myotoxin II neutralization by five different IgGs.*** Five IgGs were tested in four toxin:IgG ratios to check for *Bothrops asper* myotoxin II neutralizing abilities in an in vitro fibroblast viability assay. Assay controls included a negative control (PBS), a positive control (toxin challenge without IgG), and a non-myotoxin II binding IgG at toxin:IgG ratios of 1:3 and 0:3. Experiments were performed in triplicates and reported as means normalized to the negative control. Asterisks (*) notes statistical difference (p < 0.05) compared to the negative control (PBS). Statistics were carried out by comparing each column to the negative control (PBS) using a one-way ANOVA, followed by Dunnett's multiple comparison test.

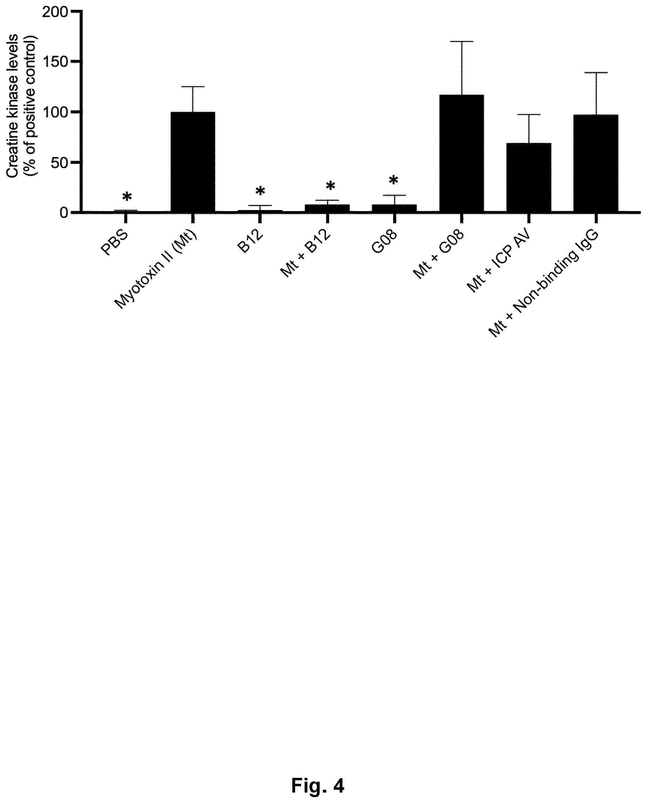

***Fig. 4******. In vivo neutralization of myotoxin II*.** The graph illustrates in vivo muscle damage caused by myotoxin II from *B*. *asper* measured as increased creatine kinase levels. Myotoxin II of *Bothrops* asperwas preincubated with antibody/antivenom for 30 minutes and then injected intramuscularly in mice. After three hours, the mice were bled, and creatine kinase levels were measured. To accommodate for assays carried out on different days, the creatine kinase levels have been normalized to the positive control (Myotoxin II). Asterisks (*) notes significant statistical difference (p < 0.05) compared to the positive control. Statistics were carried out by comparing each column to the positive control using a one-way ANOVA, followed by Dunnett's multiple comparison test. For clearer visulization, the antibody names (TPL0039_05_XXX) have been abbreviated as XXX, and ICP antivenom has been abbreviated as ICP AV.

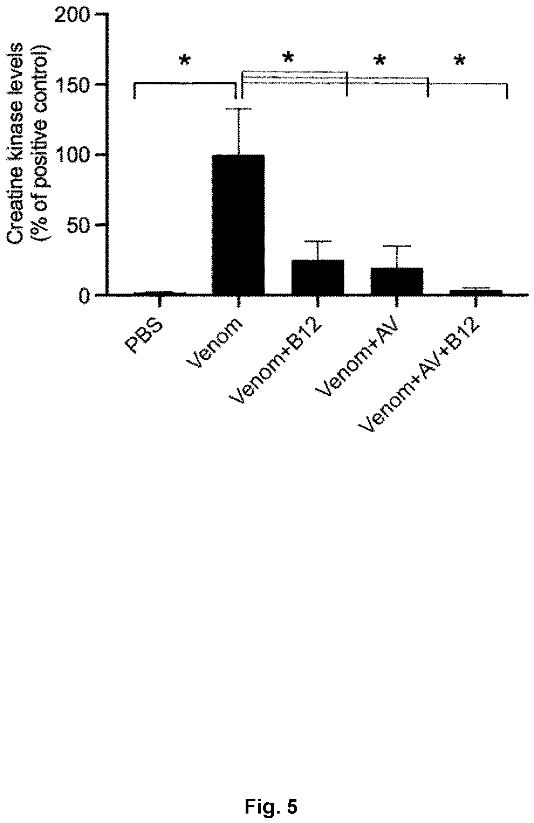

***Fig. 5******. In vivo neutralization of the muscle damaging effects caused by B. asper venom.*** The graph illustrates in vivo muscle damage caused by B. asper venom measured as increased creatine kinase levels. B. asper whole venom was preincubated with antibody/antivenom (B12/AV) for 30 minutes and then injected intramuscularly in mice. After three hours, the mice were bled, and creatine kinase levels were measured. The creatine kinase levels have been normalized to the positive control (B. asper venom) for graph consistency. Asterisks (*) notes significant statistical difference (p < 0.05) compared to the positive control. Statistics were carried out by comparing each column to the positive control using a one-way ANOVA, followed by Dunnett' multiple comparison test.

### Detailed description

### Definitions

As used herein, the singular forms "a", "an" and "the" include plural referents unless the context clearly states otherwise. Thus, for example, reference to "an antibody" includes a plurality of such antibodies.

As used herein, the term "antibody" includes full-length antibodies, antibody fragments and other antigen-binding protein constructs. The term encompasses intact antibodies that comprise at least two full-length heavy chains and two full-length light chains, as well as derivatives, variants, fragments, and mutations thereof, examples of which include Fab, Fab', F(ab')₂, and Fv fragments such as for example scFv, dsFv, or ds-scFv fragments. An "antibody" also includes domain antibodies such as nanobodies, minibodies, diabodies, camelid single domain antibodies, heavy chain variable region fragment (V_{H}), single-chain antibodies and multimers thereof, and bispecific antibodies.

As used herein the term "corresponding to" in terms of amino acids, means that following alignment between a polypeptide and a reference polypeptide, an amino acid corresponds to position X of the reference sequence if it aligns to the same position.

As used herein, the term "variant" refers to either a naturally occurring variation of a given peptide or a recombinantly prepared variation of a given peptide or protein in which one or more amino acid residues have been modified by amino acid substitution, addition, or deletion. The term "variant" may also define either a naturally occurring genetic mutant of a DNA sequence or its encoded RNA or protein product, or a recombinantly prepared variation of a DNA sequence or its encoded RNA or protein product.

As used herein, the term "altered" in terms of amino acids encompasses "deleted", "inserted" or "substituted with another amino acid". Preferably, "altered" as used herein refers to "substituted for another amino acid"

As used herein, "KD" refers to the equilibrium dissociation constant for a ligand-receptor complex. The KD value is expressed in Molar units (M) and is obtained by dividing the dissociation rate (k_{off}) by the association rate (kₒₙ). The association rate, the dissociation rate and the equilibrium dissociation constant are used to represent the binding affinity of an antibody to an antigen

As used herein, the term "Equine" refers to members of the Equidæ family and covers, but not limited to, horses, donkeys and zebras.

### Antibody capable of neutralizing myotoxin II from snake venom

The present invention relates to an antibody capable of neutralizing myotoxin II from snake venom.

The term "neutralizing" as used herein refers to an antibody which reduces or abolishes the biological activity of an antigen, such as a snake venom antigen, for instance a myotoxin, such as Myotoxin II. Thus, "neutralizing" as used herein in particular refers to the antibody being capable of reducing or abolishing the cellular toxicity of myotoxin II of SEQ ID NO:1 or a toxin sharing at least 60% identity therewith or to the antibody being capable of reducing creatine kinase levels in vivo after contact af of myotoxin II of SEQ ID NO:1 or a toxin sharing at least 60% identity therewith.

The invention relates to antibodies capable of neutralizing myotoxin II of SEQ. ID. NO: 1 or a toxin sharing at least 60% sequence identity therewith.

Myotoxin II of *Bothrops asper* or homologues thereof, such as Basic phospholipase A2 homolog 2, is one of the potent myotoxic phospholipases A₂ (PLA₂s) and PLA₂-like proteins found in the venom of several snakes, for example in the snakes of the *Bothrops* genus, such as the viper *Bothrops asper.* Thus, said toxin may be a phospholipase A₂ (PLA₂). In particular, Myotoxin II of *Bothrops asper* belong to the group of Lys49 PLA₂s and Lys49 PLA₂-like toxins. Accordingly, said toxin may in particular be a Lys49 PLA₂ or a Lys49 PLA₂-like toxin.

Several other myotoxins share a high polypeptide sequence identity with Myotoxin II from *Bothrops asper.* Thus, said toxin may in particular be a myotoxin from a snake of any of the species of the *Bothrops* genus or from species from other genera, such as *Bothrocophias, Trimeresurus, Agkistrodon, Protobothrops, Bothriechis, Cerrophidion, Gloydius, Crotalus, Deinagkistrodon, Atropoides, Cerrophidion, Lachesis, Ovophis, Echis, Vipera, Daboia.*

A first aspect of the present invention relates to an antibody capable of neutralizing myotoxin II of SEQ. ID. NO: 1 or a toxin sharing at least 60%, preferably at least 70%, yet more preferably at least 80%, such as at least 90%, for example at least 95%, such as at least 98% sequence identity therewith.

The person skilled in the art will appreciate that for example several snake genera from Central and South America, such as northern South America are known to possess a venom containing PLA₂s and PLA₂-like proteins.

In one embodiment, the toxin is from the venom of a snake from the *Bothrops* genus.

In another embodiments, the snake is selected from the group consisting of: *Bothrops asper, Bothrops alcatraz, Bothrops alternatus, Bothrops ammodytoides, Bothrops atrox, Bothrops ayerbei, Bothrops barnetti, Bothrops bilineatus, Bothrops brazili, Bothrops caribbaeus, Bothrops chloromelas. Bothrops cotiara, Bothrops diporus, Bothrops erythromelas, Bothrops fonsecai, Bothrops insularis, Bothrops itapetiningae, Bothrops jararaca, Bothrops jararacussu, Bothrops jonathani, Bothrops lanceolatus, Bothrops leucurus, Bothrops lutzi, Bothrops marajoensis, Bothrops marmoratus, Bothrops mattogrossensis, Bothrops medusa, Bothrops monsignifer, Bothrops moojeni, Bothrops muriciensis, Bothrops neuwiedi, Bothrops oligobalius, Bothrops oligolepis, Bothrops osbornei, Bothrops otavioi, Bothrops pauloensis, Bothrops pictus, Bothrops pirajai, Bothrops pubescens, Bothrops pulcher, Bothrops punctatus, Bothrops sanctaecrucis, Bothrops sazimai, Bothrops sonene, Bothrops taeniatus and Bothrops venezuelensis.*

In a further embodiment, the snake is *Bothrops asper.*

The antibodies of the present invention preferably have a high affinity for the antigen, such as a toxin, for example Myotoxin II. As used herein "binding to" refers to the formation of a non-covalent association between the antigen-binding protein and the antigen, although such binding is not necessarily reversible. The KD mentioned hereinabove can be used to measure the affinity of the binding, wherein a lower relative KD value indicates a higher affinity for the antigen.

The person skilled in the art will appreciate that the binding and affinity of the antibody can be measured by several methods known in the art. For example by immunoassays, such as ELISAs, spectroscopy-based assays, such as surface-plasmon resonance (SPR) or for example bio-layer interferometry (BLI)-based assays such as the Octet system described herein in Example 2.

In some embodiments, the antibody binds to myotoxin II of SEQ. ID. NO: 1 or to a toxin sharing at least 60% sequence identity therewith with a KD of at the most 50 nM, for example at the most 10 nM, such as at the most 1 nM, for example at the most 100 pM, such as at the most 50 pM, for example at the most 25 pM, such as at the most 10 pM, for example at the most 1 pM.

The person skilled in the art will appreciate that Myotoxin II of *Bothrops asper* is known to be cytotoxic for example to a variety of mammalian cell types, such as for example muscle cells (myotoxicity), such as skeletal muscle cells, or stromal cells, such as for example fibroblasts, as described in Example 3 herein. *B. asper* Myotoxin II is also known to possess bactericidal activity.

Accordingly, the antibody of the invention preferably is capable of reducing or even abolishing cytotoxicity of said toxin in one or more of aforementioned cell types, wherein said cytotoxicity may be determined as described in Example 3 below.

The cytotoxic and myotoxic activiy of myotoxin II of *Bothrops asper and* related myotoxins on eukaryotic cells is known in the art and is believed to involve a several-step mechanism finally leading to disruption of the integrity of the plasma membrane resulting in critical cell damage and cell death.

Without being bound by theory, the antibody of the invention may be neutralizing the cytotoxic activity of the toxin as described herein by neutralizing for instance one or more of the steps of:
- binding of the toxin to the cell membrane.
- entry of a hydrophobic molecule (e.g. a fatty acid) from the cell membrane to the hydrophobic channel of the toxin.
- allosteric activation and alignment of functional sites of one or more of the monomers (e.g. alignment of the C-terminal and membrane docking site (MDoS) side-by-side and putting the membrane-disruption sites (MDiS) in the same plane, exposed to solvent and in a symmetric position for both monomers).
- docking and/or stabilization of the functional sites of the toxin to the membrane docking site (MDoS), for instance by the interaction of charge residues with phospholipid head groups.
- activation of the membrane disrupting site (MDiS) and/or penetration of hydrophobic residues in the membrane.

In some embodiments the antibody is capable of neutralizing said toxin as determined by a method comprising the steps of:
- mixing said toxin with said antibody at a toxin-to-antibody molar ratio of no more than 1:5, for instance no more than 1:4, such as no more than 1:3, for instance no more than 1:2, such as no more than 1:1.5, for instance no more than 1:1, such as no more than 1:0.5
- incubating human cells with said mixture under conditions allowing growth of the cells,
- determining viability of said cells
wherein a viability of at least 70%, such as at least 80%, for example at least 90% compared to cells incubated under the same conditions in the absence of said toxin and said antibody is indicative of said antibody neutralising said toxin.

In other embodiments, the antibody is capable of neutralizing said toxin as determined by a method comprising the steps of:
- mixing said toxin with said antibody at a molar ratio of no more than 1:5, for instance no more than 1:4, such as no more than 1:3, for instance no more than 1:2, such as no more than 1:1.5, for instance no more than 1:1,
- administering said mixture to one or more mice,
- determining the plasma creatine kinase (CK) activity in said mice,
wherein a level of CK activity of less than 50%, preferably less than 40%, such as less than 30%, for example less than 20%, such as less than 10% compared to the CK activity in mice having received the same dosage of said toxin, but no antibody is indicative of said antibody neutralising said toxin.

The person skilled in the art will appreciate that the antigen-binding proteins recognize epitopes of the antigen. For example, an epitope can comprise or consist of a portion of a peptide (e.g. myotoxin II). An epitope can be a linear epitope, sequential epitope, or a conformational epitope. The skilled person will also appreciate that the epitope recognized by the antibody may be of importance for the neutralizing activity, for example the neutralizing activity of the antibody towards myotoxin II of SEQ ID NO:1.

In some embodiments, the antibody is capable of binding to an epitope contained between residues corresponding to amino acids 105 to 117 of the myotoxin II protein sequence as defined in SEQ. ID. No 1 or variant(s) thereof having at least 70% identity, such as at least 75% identity, for example at least 80% identity, such as at least 85% identity, for example at least 90% identity, such as at least 95% identity thereto. In particular, the antibody may be capable of binding to an epitope having the sequence of SEQ ID NO:22. Thus, the antibody preferably is capable of binding any toxin comprising SEQ ID NO:22.

### Antibody structures, sequences, and modifications

The invention relates to an antibody neutralizing myotoxin II.

In one aspect, the antibody may in particular comprise or consist of a heavy chain variable (VH) region comprising a heavy chain complementarity-determining region 3 (CDR-H3) comprising or consisting of SEQ. ID. NO: 4 , or a variant thereof, wherein in said variant up to 3 amino acids have been altered, for example wherein 2 or 1 amino acid(s) have been altered.

In one embodiment, the antibody as described herein functionally, for example in the previous section *Antibody capable of neutralizing myotoxin II from snake venom* comprises the structural sequences described in this section.

In another embodiment, the antibody comprises or consists of:
a) a heavy chain variable (VH) region comprising a heavy chain complementarity-determining region 3 (CDR-H3) comprising or consisting of any of SEQ. ID. NOs: 4, 8, 11, 12, or a variant thereof, wherein in said variant up to 3 amino acids have been altered, for example wherein 2 or 1 amino acid(s) have been altered;
   and
b) a light chain variable (VL) region comprising a light chain complementarity-determining region 3 (CDR-L3) comprising or consisting of any of SEQ. ID. NOs: 7, 10, or a variant thereof, wherein in said variant up to 3 amino acids have been altered, for example wherein 2 or 1 amino acid(s) have been altered.

In another embodiment, the antibody comprises or consists of:
a) a heavy chain variable (VH) region comprising:
   i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
   ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
   iii. a heavy chain complementarity-determining region 3 (CDR-H3) comprising or consisting of any of SEQ. ID. NO: 4, 8, 11, 12, or a variant thereof, wherein in said variant up to 3 amino acids have been altered, for example wherein 2 or 1 amino acid(s) have been altered;
   and
b) a light chain variable (VL) region comprising:
   i. a light chain complementarity-determining region 1 (CDR-L1) comprising or consisting of any of SEQ. ID. NO: 5, 13, or a variant thereof, wherein in said variant up to 3 amino acids have been altered, for example wherein 2 or 1 amino acid(s) have been altered;
   ii. a light chain complementarity-determining region 2 (CDR-L2) comprising or consisting of any of SEQ. ID. NO: 6, 9, or a variant thereof, wherein in said variant up to 3 amino acids have been altered, for example wherein 2 or 1 amino acid(s) have been altered, preferably wherein no amino acids are altered; and
   iii. a light chain complementarity-determining region 3 (CDR-L3) comprising or consisting of SEQ. ID. NO: 7, 10, or a variant thereof, wherein in said variant up to 3 amino acids have been altered, for example wherein 2 or 1 amino acid(s) have been altered.

The antibody may be the antibody defined as *TPL0039_05_B12* herein.

In an embodiment, the antibody comprises or consists of:
a) a heavy chain variable (VH) region comprising:
   i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
   ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
   iii. a heavy chain complementarity-determining region 3 (CDR-H3) comprising or consisting of SEQ. ID. NO: 4 or a variant thereof, wherein in said variant up to 3 amino acids have been altered, for example wherein 2 or 1 amino acid(s) have been altered;
   and
b) a light chain variable (VL) region comprising:
   i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
   ii. a light chain complementarity-determining region 2 (CDR-L2) comprising or consisting of SEQ. ID. NO: 6 or a variant thereof, wherein in said variant up to 3 amino acids have been altered, for example wherein 2 or 1 amino acid(s) have been altered; and
   iii. a light chain complementarity-determining region 3 (CDR-L3) comprising or consisting of SEQ. ID. NO: 7 or a variant thereof, wherein in said variant up to 3 amino acids have been altered, for example wherein 2 or 1 amino acid(s) have been altered.

The antibody may be the antibody defined as *TPL0039_05_F04* herein.

In an embodiment, the antibody comprises or consists of:
a) a heavy chain variable (VH) region comprising:
   i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
   ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
   iii. a heavy chain complementarity-determining region 3 (CDR-H3) comprising or consisting of SEQ. ID. NO: 8 or a variant thereof, wherein in said variant up to 3 amino acids have been altered, for example wherein 2 or 1 amino acid(s) have been altered;
   and
b) a light chain variable (VL) region comprising:
   i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
   ii. a light chain complementarity-determining region 2 (CDR-L2) comprising or consisting of SEQ. ID. NO: 9 or a variant thereof, wherein in said variant up to 3 amino acids have been altered, for example wherein 2 or 1 amino acid(s) have been altered; and
   iii. a light chain complementarity-determining region 3 (CDR-L3) comprising or consisting of SEQ. ID. NO: 10 or a variant thereof, wherein in said variant up to 3 amino acids have been altered, for example wherein 2 or 1 amino acid(s) have been altered.

The antibody may be the antibody defined as *TPL0039_05_G08* herein.

In an embodiment, the antibody comprises or consists of:
a) a heavy chain variable (VH) region comprising:
   i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
   ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acids have been altered; and
   iii. a heavy chain complementarity-determining region 3 (CDR-H3) comprising or consisting of SEQ. ID. NO: 11 or a variant thereof, wherein in said variant up to 3 amino acids have been altered, for example wherein 2 or 1 amino acids have been altered;
   and
b) a light chain variable (VL) region comprising:
   i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
   ii. a light chain complementarity-determining region 2 (CDR-L2) comprising or consisting of SEQ. ID. NO: 6 or a variant thereof, wherein in said variant up to 3 amino acids have been altered, for example wherein 2 or 1 amino acid(s) have been altered; and
   iii. a light chain complementarity-determining region 3 (CDR-L3) comprising or consisting of SEQ. ID. NO: 10 or a variant thereof, wherein in said variant up to 3 amino acids have been altered , for example wherein 2 or 1 amino acid(s) have been altered .

The antibody may be the antibody defined as *TPL0039_05_B04* herein.

In an embodiment, the antibody comprises or consists of:
a) a heavy chain variable (VH) region comprising:
   i. a heavy chain complementarity-determining region 1 (CDR-H1) comprising or consisting of SEQ. ID. NO: 2 or a variant thereof, wherein in said variant up to 3 amino acids have been altered , for example wherein 2 or 1 amino acid(s) have been altered ;
   ii. a heavy chain complementarity-determining region 2 (CDR-H2) comprising or consisting of SEQ. ID. NO: 3 or a variant thereof, wherein in said variant up to 3 amino acids have been altered , for example wherein 2 or 1 amino acid(s) have been altered ; and
   iii. a heavy chain complementarity-determining region 3 (CDR-H3) comprising or consisting of SEQ. ID. NO: 12 or a variant thereof, wherein in said variant up to 3 amino acids have been altered , for example wherein 2 or 1 amino acids have been altered ;
   and
b) a light chain variable (VL) region comprising:
   i. a light chain complementarity-determining region 1 (CDR-L1) comprising or consisting of SEQ. ID. NO: 13 or a variant thereof, wherein in said variant up to 3 amino acids have been altered , for example wherein 2 or 1 amino acid(s) have been altered ;
   ii. a light chain complementarity-determining region 2 (CDR-L2) comprising or consisting of SEQ. ID. NO: 6 or a variant thereof, wherein in said variant up to 3 amino acids have been altered , for example wherein 2 or 1 amino acid(s) have been altered ; and
   iii. a light chain complementarity-determining region 3 (CDR-L3) comprising or consisting of SEQ. ID. NO: 10 or a variant thereof, wherein in said variant up to 3 amino acids have been altered , for example wherein 2 or 1 amino acid(s) have been altered .

The antibody may be the antibody defined as *TPL0039_05_A03* herein.

In an embodiment, the antibody comprises or consists of:
a) a heavy chain variable (VH) region comprising:
   i. a heavy chain complementarity-determining region 1 (CDR-H1) comprising or consisting of SEQ. ID. NO: 2 or a variant thereof, wherein in said variant up to 3 amino acids have been altered , for example wherein 2 or 1 amino acid(s) have been altered ;
   ii. a heavy chain complementarity-determining region 2 (CDR-H2) comprising or consisting of SEQ. ID. NO: 3 or a variant thereof, wherein in said variant up to 3 amino acids have been altered , for example wherein 2 or 1 amino acid(s) have been altered ; and
   iii. a heavy chain complementarity-determining region 3 (CDR-H3) comprising or consisting of SEQ. ID. NO: 23 or a variant thereof, wherein in said variant up to 3 amino acids have been altered , for example wherein 2 or 1 amino acids have been altered ;
   and
b) a light chain variable (VL) region comprising:
   i. a light chain complementarity-determining region 1 (CDR-L1) comprising or consisting of SEQ. ID. NO: 5 or a variant thereof, wherein in said variant up to 3 amino acids have been altered , for example wherein 2 or 1 amino acid(s) have been altered ;
   ii. a light chain complementarity-determining region 2 (CDR-L2) comprising or consisting of SEQ. ID. NO: 9; and
   iii. a light chain complementarity-determining region 3 (CDR-L3) comprising or consisting of SEQ. ID. NO: 7 or a variant thereof, wherein in said variant up to 3 amino acids have been altered , for example wherein 2 or 1 amino acid(s) have been altered .

In an embodiment, the antibody comprises or consists of:
a) a heavy chain variable (VH) region comprising or consisting of any of SEQ. ID. NO: 14, 16, 18, 20 or a variant thereof, wherein in said variant up to 5 amino acids have been altered, for example wherein 4, 3, 2, or 1 amino acid(s) have been altered; and
b) a light chain variable (VL) region comprising or consisting of any of SEQ. ID. NO: 15, 17, 19, 21 or a variant thereof, wherein in said variant up to 5 amino acids have been altered, for example wherein 4, 3, 2, or 1 amino acid(s) have been altered.

In an embodiment, the antibody comprises or consists of:
a) a heavy chain variable (VH) region comprising or consisting of SEQ. ID. NO: 14, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: 15, or a variant thereof, wherein in said variant up to 5 amino acids have been altered, for example wherein 4, 3, 2, or 1 amino acid(s) have been altered.

In an embodiment, the antibody comprises or consists of:
a) a heavy chain variable (VH) region comprising or consisting of SEQ. ID. NO: 16, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: 17, or a variant thereof, wherein in said variant up to 5 amino acids have been altered , for example wherein 4, 3, 2, or 1 amino acid(s) have been altered.

In an embodiment, the antibody comprises or consists of:
a) a heavy chain variable (VH) region comprising or consisting of SEQ. ID. NO: 18, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: 19, or a variant thereof, wherein in said variant up to 5 amino acids have been altered , for example wherein 4, 3, 2, or 1 amino acid(s) have been altered.

In an embodiment, the antibody comprises or consists of:
a) a heavy chain variable (VH) region comprising or consisting of SEQ. ID. NO: 20, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: : 21, or a variant thereof, wherein in said variant up to 5 amino acids have been altered , for example wherein 4, 3, 2, or 1 amino acid(s) have been altered.

In some embodiments, the one or more amino acids of the antibody that have been altered for another amino acid is (are) an amino acid(s) not part of the CDR as defined herein. In preferred embodiments, however, the antigen-binding proteins of the invention comprise the specific CDR sequences provided herein.

Eventhough the invention in its broadest scope comprises antibodies comprising variants of the heavy and light variable regions and variants of the CDRs thereof, it is preferred that the antibodies comprise one or more of the exact sequences of the heavy and light variable regions and of the CDRs thereof as set forth in any of SEQ. ID. NOs 2 to 21.

As used herein "signal peptide" refers to a short (less than 60 amino acids, for example, 3 to 60 amino acids) polypeptide present on precursor proteins (typically at the N terminus), and which is typically absent from the mature protein. The signal peptide is typically rich in hydrophobic amino acids. The signal peptide directs the transport and/or secretion of the translated protein through the membrane. Signal peptides may also be called targeting signals, transit peptides, localization signals, or signal sequences. For example, the signal sequence may be a co-translational or post-translational signal peptide.

The skilled person will appreciate that it may be beneficial to improve the secretion of antigen-binding protein during its production in host cell lines, for instance during its production in the Chinese hamster ovary (CHO) cell line. Improving the secretion efficiency of the recombinant antigen-binding protein may improve its final yield.

In some embodiments, the VL and/or the VH region of the antigen-binding protein further comprises an N-terminal signal peptide sequence.

In one embodiment, the antibody comprises an immunoglobulin constant region.

In preferred embodiments, the antibody is selected from the group consisting of a full-length antibody, a Fab fragment, a F(ab') fragment, a F(ab')₂ fragment, an scFv, a diabody, and a triabody.

The antibody may be a monoclonal antibody, preferably a human antibody or a chimeric antibody. The antibody may be from recombinant sources and/or produced in transgenic animals. The term " antibody" as used herein also includes includes full-length antibodies, antibody fragments and other antigen-binding protein constructs. The term encompasses intact antibodies that comprise at least two full-length heavy chains and two full-length light chains, as well as derivatives, variants, fragments, and mutations thereof, examples of which include Fab, Fab', F(ab')2, and Fv fragments such as for example scFv, dsFv, or ds-scFv fragments. An "antibody" also includes domain antibodies such as nanobodies, minibodies, diabodies, camelid single domain antibodies, heavy chain variable region fragment (VH), single-chain antibodies and multimers thereof, and bispecific antibodies. Antibodies can be fragmented using conventional techniques. For example, F(ab')₂ fragments can be generated by treating the antibody with pepsin. The resulting F(ab')₂ fragment can be treated to reduce disulfide bridges to produce Fab' fragments. Papain digestion can lead to the formation of Fab fragments. Fab, Fab' and F(ab')₂, scFv, dsFv, ds-scFv, dimers, minibodies, diabodies, bispecific antibody fragments, and other fragments can also be synthesized by recombinant techniques. The antibodies are optionally in any useful isotype, including IgM, IgA, such as IgA1, IgA2, IgD, IgE or IgG, such as IgG1, IgG2, IgG3 or IgG4.

Thus, in some embodiments, the antibody comprises an immunoglobulin constant region selected from IgG, IgM, IgA, IgD, or IgE.

In some other embodiments, the antibody comprises an immunoglobulin constant region selected from IgG and IgA.

In other embodiments, the antibody comprises an immunoglobulin constant region selected from IgG1, IgA1, and IgA2.

In another embodiment, the antibody comprises an immunoglobulin constant region selected from IgG1, IgG2, IgG3, and IgG4.

In preferred embodiments, the antibody is a monoclonal antibody.

In yet other embodiments, the antibody is a human antibody or a chimeric antibody.

The antibodies of the invention preferably have advantageous biophysical and pharmacokinetic properties for good efficacy, and manufacturability.

The skilled person will appreciate that antibodies may be modified for improving their stability *in vitro* and/or *in vivo.* For example the antibodies may be modified to improve circulating half-life, such as for example when used *in vivo.* Increased antibody half-life may improve for instance its distribution, efficacy and duration of its functional effect. Such modifications include conjugation with molecules providing a higher molecular weight and/or hydrodynamic radius to the antibody, such as PEGylation, polysialylation or conjugation to any other protein or small molecule known to be suitable by the skilled person. The modification may also be associated with increased evasion from the immune system, thereby also increasing the circulatory time *in vivo.* Other approaches which may improve the antibody half-life include engineering of the Fc region, such as for example a mutation of the Fc region associated with reduced antigen-binding turnover *in vivo.* The antibody, in particular recombinant antibodies, may also for instance be modified to improve their biological function and efficacy. The skilled person will appreciate that several post-translational modifications, such as glycosylations may have a critical impact on the efficacy of antibodies, for example impacting their antigen binding capabilities or effector functions. For instance the glycosylations may be glycosylations of the Fc region or the Fab of the antibody, such as one or more N-glycosylations.

Thus in some embodiments, the antibody has one or more modification(s) selected from the group consisting of PEGylation, polysialylation, Fc region mutation, and N-glycosylation.

The skilled person will appreciate that it may be beneficial to be able to detect and follow the antibody for some applications. For instance conjugating the antibody with a detection label, or detection agent, may be useful for diagnostic purposes, imaging purposes, multiplexing assays and other assays known to the skilled person. The label may be a primary label, a secondary label or any combinations thereof known to be suitable by the skilled person.

Thus, in one embodiment, the antibody comprises a detection label.

In some applications, for instance spectroscopic assays, colorimetric assays, and light microscopy, it may be beneficial to use a colored or a luminescent dye, for instance chromophores and phosphors or any dye known to be suitable by the skilled person. Other applications such as for example fluorescent microscopic applications, flow cytometry and fluorescence-activated cell sorting (FACS), hybridization assays, or any immunoassays known to the skilled person may require or benefit from an antibody comprising a fluorescent label or moiety. The detection label may also be a magnetic or paramagnetic label, for instance for magnetic separation application or immunoassays known to the skilled person.

In some embodiments, the antibody label is selected from the group consisting of a colorimetric, a fluorescent, a luminescent, a magnetic, and a paramagnetic label.

The skilled person will also appreciate that other labels which can be used for instance for separation and purification, as well as for example detection in immunoassays, include the biotin-streptavidin pair. The antibody may be biotinylated for instance for avidin (or streptavidin) detection.

In some embodiments, the detection label on the antibody is biotin.

For other applications, such as electron microscopy, flow cytometry and lateral flow immunoassays, the skilled person will appreciate that conjugation with gold nanoparticles can be used for the detection and/or visualisation of the presence or binding or the antibody.

In some embodiments of the present invention, the detection label is a gold nanoparticle

### Preparation of the antibody

The antibody according to the invention may be prepared by any useful method. In one embodiment, the antibodies are prepared by recombinant methods comprising cultivating a host cell comprising a nucleic acid encoding the antigen-binding protein under control of an element directing expression in said host cell.

The antibodies are preferably identified using phase display, such as using the library as described in (Schofield et al., 2007), although other methods known in the art may also be employed. More preferably, the antigen-binding protein is identified using the methods described in Example 1 below.

The antibodies may for instance be developed from scFVs clones binding to the antigen of interest, such as Myotoxin II of *Bothrops asper as* described herein. scFV clones may be further converted to larger antibody fragments, such as Fab, or to full length antibodies, such as IgGs, for instance by cloning the variable domains into suitable expression vectors known in the art. More preferably, the antibodies are developed using the methods described in Example 2 below.

### Pharmaceutical compositions and combinations of antigen-binding proteins with other agents

Another aspect of the present invention relates to a pharmaceutical composition comprising an antibody as defined herein, and a pharmaceutically acceptable diluent, carrier and/or excipient.

The antibodies of the present invention may be combined in formulations comprising other antigen-binding protein(s) capable of binding to, blocking or neutralizing one or more snake venom antigen(s). The other antigen-binding protein(s) capable of binding to, blocking or neutralizing one or more snake venom antigen(s) may be for instance existing commercial antigen-binding protein-based antivenoms, such as the antivenoms listed in (Laustsen et al., 2016), Supplementary Table 1.

In other embodiments, the pharmaceutical composition further comprises one or more additional antigen-binding protein(s) capable of binding to, blocking or neutralizing one or more snake venom antigen(s).

A further aspect of the present invention relates to a kit-of-parts comprising:
a) an antibody as described herein; and
b) another agent suitable for the treatment of snake envenomation.

### Antigen-binding proteins

In some embodiments, the other agent used for the treatment of snake envenomation in the kit-of-parts is one or more antigen-binding protein(s) capable of binding to, blocking, and/or neutralizing one or more snake venom antigen(s) selected from the group consisting of: IgG, IgM, scFv, F(ab)₂, Fab, Fab', V_{H}H antigen-binding protein(s) and fragments thereof.

The skilled person will know that antivenom preparations, containing antigen-binding protein(s) capable of neutralizing one or more snake venom antigen(s) are traditionally obtained from hyperimmune equine, ovine, rabbit or camelid organisms. Several equine, ovine, rabbit or camelid-plasma derived antigen-binding proteins, or fragments thereof, may thus be combined with the antigen-binding protein as described herein.

In further embodiments, the one or more additional antigen-binding protein(s) capable of neutralizing one or more snake venom antigen(s) is (are) selected from the group consisting of: Equine immunoglobulin(s) against snake venom, such as Equine IgG(s) or Equine Fab or Equine F(ab')₂ fragment(s) against snake venom, Ovine immunoglobulin(s) against snake venom, such as Ovine IgG(s) or Ovine Fab or Ovine F(ab')₂ fragment(s) against snake venom, Rabbit immunoglobulin(s) against snake venom, such as Rabbit IgG(s) or Rabbit Fab or Rabbit F(ab')₂ fragment(s) against snake venom, and Camelids immunoglobulin(s) against snake venom, such as Camelids IgG(s) or Camelids Fab or Camelids F(ab')₂fragment(s) against snake venom.

### Neutralizing proteins

Another type of agents which may be used for the treatment of snake envenomations are neutralizing peptides and protein inhibitors, for instance as disclosed in (Laustsen et al., 2016).

In some other embodiments, the other agent used for the treatment of snake envenomation is one or more protein(s) displaying neutralization potential against snake venoms.

In preferred embodiments, the one or more protein(s) displaying neutralization potential against snake venoms is (are) selected from the group consisting of: a PLA₂ inhibitor, a matrix metalloproteinase (MMP) inhibitor, a snake venom metalloproteinase inhibitor (SVMP), a Kunitz-type trypsin inhibitor, an antimyotoxic factor, an antimyonecrotic factor, an antineurotoxic factor, an antihæmmoragic factor, an antioedematogenic factor and a glycoprotein, such as acid-a1-glycoprotein.

### Small molecules

A further type of agents used in the treatment of snake envenomation are small molecules, for instance as described in (Laustsen et al., 2016).

In some embodiments, the other agent used for the treatment of snake envenomation is one or more small molecule(s) with inhibitory effect against snake toxins.

In further embodiments, the one or more small molecule(s) with inhibitory effect against snake toxins is (are) selected from the group consisting of: polyanions, polyphenolic compounds, and metalloproteinase inhibitors.

In preferred embodiments, the one or more small molecule(s) with inhibitory effect against snake toxins is (are) selected from the list consisting of : Suramin, Heparin, Wedelolcatone, Coumestrol, Glycyrrhizin, Fucoidan, Apigenin analogue, AG-3340, Aristolochic acid, Rosmarinic acid, Imidazopyridine, BAPTA, TPEN, EDTA, DMPS, dimercaprol, BAY-129566, Marimastat, CGS-27023A, Batimastat, Nafamostat, MV 8612 from Mandevilla velutina, MV 8608 from Mandevilla velutina, Koninginin, 4-nerolidylcatechol, Anisodamine, 12-methoxy-4-methylvoachalotine (MMV), Quinonoid xanthene ehretianone, Benzoylsalireposide, Salireposide, Chlorogenic acid, Caffeic acid, 2-hydroxy-4-methoxy benzoic acid, and Varespladib.

### Treatment of snake envenomation

Another aspect of the present invention relates to an antibody as described herein, a pharmaceutical composition as described herein or a kit-of-parts as described herein for use in a method of treatment of snake envenomation in a subject. Said snake may be any of the snakes described herein above in the section *Antibody capable of neutralizing myotoxin II from snake venom.*

In a preferred embodiment, the antibody, the pharmaceutical composition or the kit-of-parts for the use as described herein prevents or delay venom-induced lethality.

In other embodiments, the antibody, the pharmaceutical composition or the kit-of-parts for the use as described herein, prevents or delays envenomation symptoms.

The skilled person will appreciate that the main effect of snake envenomation, in particular snake envenomation by a venom containing PLA₂s or PLA₂-like proteins, such as myotoxin II, is muscle and/or tissue damage. Several other direct and indirect symptoms can occur as a result to snake envenomation.

In some embodiments, the envenomation symptoms comprise pain, oozing from the puncture wounds, local swelling, bruising, blisters, numbness, mild fever, headache, tissue damage, muscle damage, kidney injury, dermonecrosis, bleeding from the nose and gums, hemoptysis, gastrointestinal bleeding, hematuria, hypotension, nausea, vomiting, impaired consciousness and tenderness of the spleen.

The skilled person will appreciate that Myotoxin II of *Bothrops asper* and other myotoxic PLA₂s are responsible for serious clinical effects, such as tissue loss, disability, and amputation.

In other embodiments, the antibody, the pharmaceutical composition or the kit-of-parts prevents or delays necrosis and/or gangrene.

The skilled person will also appreciate that myonecrosis can be assessed indirectly by measuring the level of enzymes such as for example creatine kinase, lactate dehydrogenase, aldolase, myoglobin, troponin, aspartate aminotransferase, and/or carbonic anhydrase CAIII.

In another embodiment, the antibody, the pharmaceutical composition or the kit-of-parts prevents the increase or reduces myonecrosis as determined for example by the level of creatine kinase in the subject following the envenomation.

In preferred embodiments, the antibody, the pharmaceutical composition or the kit-of-parts as described herein is administered parenterally or pulmonarily.

In yet other embodiments, the parenteral administration of the antibody, the pharmaceutical composition or the kit-of-parts is intravenous, intramuscular and/or subcutaneous.

The skilled person will appreciate that antivenoms should ideally be provided to the subject in need thereof as soon as possible after the bite. Antivenoms may however not be available close to the location of the envenomation event, in particular when the snake bite occurs in remote geographical areas, for example in geographical areas with limited vehicle access, complicated logistics, and/or scarce healthcare center presence or healthcare resources. It may thus be of great benefit that the antigen-binding protein, the pharmaceutical composition or the kit-of-parts for use in the treatment of snake envenomation can be administered shortly after the bite but also may have a preventing or delaying action on the symptoms or a fortiori on lethality when administered relatively longer after the bite.

In some embodiments, the antibody, the pharmaceutical composition or the kit-of-parts is administered no more than 5 min after envenomation, such as no more than 10 min after envenomation, for instance no more than 15 min after envenomation, such as no more than 20 min after envenomation, for instance no more than 30 min after envenomation, such as no more than 45 min after envenomation, for instance no more than 60 min after envenomation, such as no more than 90 min after envenomation, for instance no more than 120 min after envenomation, such as no more than 180 min after envenomation, for instance no more than 240 min after envenomation, such as no more than 6h after envenomation, for instance no more than 12h after envenomation, such as no more than 24h after envenomation, for instance no more than 48h after envenomation, such as no more than 72h after envenomation, for instance no more than 96h after envenomation, such as no more than 120h after envenomation for instance no more than 144h after envenomation.

In another aspect, the present invention relates to a method of treating snake envenomation which comprises administering to a subject an effective amount of the antibody as described herein, the composition as described herein, or the kit-of-parts as described herein.

A further aspect of the present invention relates to the use of the antibody as described herein, the composition as described herein, or the kit-of-parts as described herein, for the manufacture of a medicament for the treatment of snake envenomation.

### Diagnosis of snake envenomation

The antibody of the present invention may be used for detection, diagnosis, and diagnostics purposes, for example in clinical settings. The antigen-binding protein may be used for instance as part of a companion diagnostic kit to support for example the clinical management of snakebites and identify therapeutic options.

Another aspect of the invention relates to a kit for detection and/or quantification of snake venom in a sample comprising:
- one or more antibodies, such as two antibodies, wherein at least one of said antibodies is as described herein; and
- means for detection of a complex comprising said one or more antibodies bound to snake venom or toxins.

In some embodiments, the kit further comprises means for acquiring or isolating a sample from a subject.

In preferred embodiments, the sample is a blood sample, a urine sample or a smear of the bite site/wound.

In further embodiments, the blood sample is a plasma blood sample.

The skilled person will appreciate that the kit may be an immunoassay, such as a method for detecting one or more analytes, such as snake venoms, or fractions thereof, for example PLA₂S or PLA₂-like proteins such as myotoxin II. Typically, the immunoassay may be a method based on the specific binding of the antibodies of the present invention to PLA₂S or PLA₂-like proteins suchs as myotoxin II. Suitable immunoassays include ELISA (enzyme-linked immunosorbent assay), indirect ELISA, sandwich ELISA, competitive ELISA, multiplex ELISA, radioimmunoassay (RIA), ELISPOT technologies, and other similar techniques known in the art.

In a further embodiment of the present invention, the kit is an immunoassay, such as a lateral flow assay, an ELISA, an electrochemical impedance spectroscopy-based assay, a Bio-layer Interferometry (BLI)-based assay or a turbidimetric immunoassay.

Another aspect of the invention relates to an antigen-binding protein as described herein for use in the diagnosis of snake envenomation in a subject.

The antigen-binding protein may be used for instance for the diagnosis of snake envenomation with a venom containing one or more PLA₂S or PLA₂-like proteins such as myotoxin II.

A further aspect of the present invention relates to the use of an antibody as described herein in an *in vitro* method for detection and/or diagnosis of snake envenomation in a subject.

Another aspect of the present invention relates to a method for detecting snake venom in a sample, said method comprising:
a) providing a sample for analysis;
b) contacting the sample with one or more antibodies as defined herein to said sample; and
c) detecting a complex comprising said one or more antibodies bound to a snake antigen.

In some embodiments, the method is an *in vitro* method.

In further embodiments, the detection of the complex is performed by an immunoassay, such as a lateral flow assay, an ELISA, an electrochemical impedance spectroscopy-based assay, a Bio-layer Interferometry (BLI)-based assay or a turbidimetric immunoassay.

### Organisms subject to snake envenomation

The person skilled in the art will appreciate that the use of antivenoms may not be restricted to humans but can also be used on non-human animals, such as for instance pets, cattle, farm animals, or ornamental animals.

In one embodiment of the present invention the subject of the antibody, the pharmaceutical composition or the kit-of-parts for the use as described herein, the kit as described herein, or the antibody for use as described herein is a human or a non-human animal.

In a further embodiment, the non-human animal subject belongs to the family of Equidae, Bovidae, Canidae, or Felidae.

In some embodiments, the non-human animal is a horse, a cow, an ox, a dog, or a cat.

### Examples

### Example 1 : Phage display selection and initial screening of monoclonal scFvs

### Material and methods

### Purification of myotoxin II

Myotoxin II (Uniprot P24605) was purified from the venom of Bothrops asper by cation-exchange chromatography on CM-Sephadex C25, followed by reverse-phase HPLC on C18, as described previously (Lomonte et al., 1989, Mora-Obando et al., 2014).

### Biotinylation of myotoxin II from B. asper

Lyophilized myotoxin II was solubilized in phosphate buffered saline (PBS) and mixed with biotin linked to N-hydroxysuccinide (NHS) via a PEG4 linker (EZ-LinkTM NHS-PEG4-Biotin, Thermo Scientific, #A39259) in a molar ratio of 1:1.5 (toxin:biotin) according to Ahmadi et al., 2020. Purification of the biotinylated toxins was also carried out by buffer exchange according to Ahmadi et al., 2020.

### Phage display selection and assessment of polyclonal output

For phage display selection, the IONTAS phage display library λ was employed, which is a human antibody phage display library with a clonal diversity of 1.6 x 1010. The displayed antibodies come in the form of scFvs and have been constructed from B lymphocytes collected from 43 non-immunized human donors (Schofield et al. 2007). Phage display selection was carried out as earlier described (Schofield et al. 2007), with the following modifications described briefly: For selections, biotinylated myotoxin II from *B. asper*(10 µg/mL) was indirectly immobilized using streptavidin-coated (10 µg/mL) Maxisorp vials, employing a streptavidin-biotin capture system. In the second and third selection round, neutravidin was used instead of streptavidin to prevent accumulation of streptavidin-recognizing antibodies. Elution in the third round was carried out by incubating with a 10 mM glycine-HCL solution at pH 6 for 15 minutes instead of eluting with trypsin.

After three rounds of phage display selection, antigen specificity of the phage output was evaluated. This was carried out according to Føns et al., 2020, using a similar protocol to the phage display selections. The phage output was purified utilizing polyethylene glycol precipitation (Schofield et al., 2007), and binding was tested against Maxisorp vials coated with either (i) biotinylated myotoxin II from*Bothrops asper* indirectly immobilized using streptavidin, (ii) streptavidin, or (iii) 3% (w/v) skimmed milk in PBS (M-PBS). Biotinylated B. *asper* myotoxin II and streptavidin were used at a concentration of 10 µg/mL.

### Sub-cloning, primary screening, and sequencing of scFvs

To facilitate expression of soluble scFvs, the scFv genes from the third selection round were sub-cloned from the phage display vector (pIONTAS1) into the pSANG10-3F expression vector using Ncol and Notl restriction endonucleases. The expression vectors were then transformed into E. coli BL21(DE3) cells (New England Biolabs), following protocols from Martin et al., 2006. Individual scFv-producing monoclonal colonies (276 colonies) were picked and expressed in 96-well format using auto-induction media (Studier et al., 2005). To determine the binding of the scFvs to myotoxin II, a monoclonal scFv ELISA was carried out. 96-well Maxisorp plates with biotinylated B. *asper* myotoxin II (5 µg/mL) indirectly immobilized using streptavidin (10 µg/mL) were used. For detection of binding, a 1:20,000 dilution of Anti-FLAG M2-Peroxidase (HRP) (Sigma Aldrich, #A8592) antibody in 3% M-PBS and an OPD solution (Sigma-Aldrich, P5412) was used as substrate according to the manufacturer's protocol. A biotinylated HPLC fraction from Naja mossambica containing PLA2 was included as a negative control. Further controls included wells coated with streptavidin/neutravidin (10 µg/mL) and 3% M-PBS. Results were measured as absorbance at 492 nm using an Epoch spectrophotometer from Biotek (15020518) with the Gen5 2.07 software.

The genes encoding scFvs binding specifically to B. *asper* myotoxin II with absorbance (492 nm) values above a pre-set threshold of 0.2 were sequenced (Eurofins and Macrogen genomics sequencing service) using the S10b primer (GGCTTTGTTAGCAGCCGGATCTCA). The antibody framework and CDR regions were annotated and analysed using the Jalview 2.10.5 program and Abysis.org to identify unique scFvs.

### Results

Using the IONTAS antibody phage display library λ (Schofield et al., 2007), single-chain variable fragment (scFv) displaying phages were selected against myotoxin II from B. *asper.* Three rounds of panning against B. *asper* myotoxin II were carried out to enrich for phages displaying scFvs with specificity to myotoxin II. To assess the success of the selection, the polyclonal outputs were investigated for their binding to B. *asper* myotoxin II by determining the number of colony-forming units (CFU) after each panning. Following the third round of panning, the specificity of the scFv-displaying phages was tested and confirmed (Fig. 1A, 1B).

Upon confirmation of successful accumulation of B. *asper* myotoxin II binders, DNA encoding the scFvs was amplified from the polyclonal phage outputs, and the genes were subcloned and expressed. Characterization of 276 scFv-producing monoclonal cultures using ELISA yielded 188 scFvs binding to *B*. *asper* myotoxin II (Fig. 1C). These 188 scFvs were screened for their specificity to myotoxin II by including milk proteins, streptavidin, neutravidin, and a PLA2 from Naja mossambica as controls. Results demonstrated that all 188 scFvs of interest were binding specifically to *B*. *asper* myotoxin II and not to any of the controls (Data not shown). The genes encoding the VH and VL for all clones were sequenced, revealing 123 scFv sequences with unique CDR regions, of which 59 showed unique VH CDR3 regions (Fig. 1D).

### Conclusions

scFVs clones specifically binding to *B*. *asper* myotoxin II could be identified and sequenced by the methods described herein, with several scFVs displaying unique CDR regions including unique VH CDR3 regions.

### Example 2: Ranking of scFv binding and affinity assessment of IgG-reformatted clones

### Material and methods

### Expression-Normalized Capture (ENC) assay

123 unique scFvs were tested in an ENC assay, which was carried out as earlier described (Laustsen et al., 2018), with the exception of using an antigen molar concentration of 10 nM.

### Conversion from scFv to antigen-binding fragment (Fab) format

VL and VH sequences from the scFvs were codon optimized for expression in human cells and synthesized by Integrated DNA Technologies (IDT). The VL was cloned using Nhel and Notl, and the VH was cloned using Ncol and Xhol restriction endonucleases into mammalian expression vector pINT12 containing the gene for the CH1 antibody domain. The CL antibody domain sequence and heavy chain promoter were cloned in a stuffer fragment using Notl and Ncol restriction endonucleases.

Following ligation of the four fragments, the construct was transformed into DH10B E. coli cells and sequence verified. For sequencing, pINT VH Seq Fwd (TGGAGCTGTATCATCCTCTTCTTGG) and pINT VL lambda seq R (ACTCCGGCTTTCACTGGGGAGCTGTCTGCCTTC) were used.

### Expression, purification, and buffer exchange of Fab

The Fabs were expressed by transient transfection of the constructed expression plasmid in Expi293FTM cells (Life TechnologiesTM, A14527) using the Expi293TM transfection system (Life TechnologiesTM, A14525) according to the manufacturer's instructions. The Fabs were purified using CH1 CaptureSelectTM IgG-CH1 Affinity Matrix (Thermo scientific, 194320010) resin beads, and Proteus '1 step batch' midi spin column (Generon, NB-45-00080). The Fabs were eluted using 0.2 M Glycine at pH 2.6, and the eluate was immediately neutralized using 1 M Tris buffer at pH 8.0. The eluted Fabs were buffer exchanged into PBS using IllustraTM NAPTM 10 columns according to manufacturer's instructions.

### Conversion from scFv to IgG format

Antibody variable domains were converted from scFv to IgG format prior to expression in CHO-S cells. The variable chains (VL and VH) were extracted from the pSANG10-3F vector by PCR and were cloned into a single expression vector using the NEBuilder^{®} cloning technique. The expression vector contained the constant domain sequences of the respective human IgG heavy chain (with LALA (Xu et al., 2000) and YTE (Acqua et al., 2002) mutation) and human kappa light chain. After cloning, expression vectors were sequence verified (Eurofins), and plasmids were purified using NucleoBond Xtra Midi EF (Macherey-Nagel) according to the manufacturer's instructions. A CHO-S cell line with pre-established landing pad suitable for recombinase-mediated cassette exchange with the IgG expression vector were cultivated in CD CHO medium (Gibco), supplemented with 8 mM L-Glutamine (Thermo Fisher Scientific) and 2 uL/mL anti-dumping agent (Gibco) at 37°C, 5% CO2 at 120 rpm (shaking diameter 25 mm). The cell line was transfected with IgG expression vector and Cre-recombinase vector in 3:1 ratio (w:w) in 6-well plates (BD-biosciences) at a concentration of 106 cells/mL using FreeStyle MAX transfection reagent (Thermo Fischer Scientific) according to the manufacturer's recommendation. Stable cell pools were generated by adding 5 µg/mL blasticidin five days post-transfection, continuing until cell death of untransfected cells and complete recovery of transfected cells (>95% viability). IgG producing cell pools were seeded at 3x105 cells/mL in FortiCHO or CD CHO medium supplemented with 8 mM glutamine and 2 µL/mL anti-clumping reagent. The pools were cultivated in Batch mode (CD CHO) for 5 days or in Fed-batch mode (Forti-CHO) for 7 to maximum 13 days, feeding glutamine, glucose, and feeding supplements cell boost 7a and 7b (VWR) starting on either day 3, 4, or 5. Cultures were harvested by centrifuging the cell suspension at 300 g for 10 minutes followed by 4700 g for 15 minutes, removing cells and cell debris. Clarified supernatants were stored at -80°C until the time point of purification.

### Purification of IgGs

Supernatants were thawed overnight, either at 4 or 10 oC, cleared once more by centrifugation at 4500 g for 15 minutes, and loaded on a column packed with 25 mL MabSelect PrismA proteinA chromatography resin (Cytiva). An aqueous solution of 20 mM sodium phosphate and 150 mM NaCl (pH 7.2) was used for equilibration and washing of the column. Elution was performed with 0.1 M sodium citrate (pH 3). Elution fractions were neutralized by having 1 M Tris (pH 9) in the collection plate, using 0.2 mL per 1 mL of elution fraction. Fractions of interest were pooled and loaded on a HiPrep 26/10 desalting column for exchange of elution buffer to PBS. Fractions containing the protein were concentrated by centrifugal filtration using an Amicon^{®} Ultra-15 centrifugal filter unit (30 kDa NMWL), aiming for a minimum concentration of 20 mg/mL. Upon collection from the centrifugal filter unit, protein solutions were filter sterilized. The final concentration was determined by measuring the absorbance at 280 nm on a Nanodrop2000 instrument. Proteins were stored at 4 oC.

### Monoclonal IgG ELISA

A monoclonal IgG ELISA was set up to test whether the binding properties of the scFv were retained through reformatting to the IgG1 format. The monoclonal IgG ELISA was carried out according to Føns et al., 2020, with the exception that the biotinylated myotoxin II from *B*. asperwas immobilized in streptavidin coated (10 µg/mL) wells and supernatants of IgG expression were used instead of purified IgGs. Absorbance was measured at 450 nm in a Multiskan FC Microplate Photometer (Thermo Scientific).

### Octet measurement

1x Kinetic Buffer (KB, ForteBio) prepared in PBS was used as the running buffer in the octet experiment. Immediately prior to the assay, the streptavidin (SAX) biosensors were pre-wet for 10 min in 1X KB. Kinetic assay was performed by first capturing biotinylated-MII using SAX biosensors followed by a 120 s baseline step in 1X KB. The Mil-captured biosensors were then dipped in wells containing increasing concentrations of Fab or IgGs - 0nM, 1 nM, 10 nM and 100 nM for a 600 s of association step, followed by a dissociation step in 1X KB for 600 s. The experiment was performed at 30°C with a shaking at 1000 rpm. FroteBio's data analysis software was used to fit the curve using 1:1 binding model to determine the ka, kd and KD (except for TPL0039_05_G08 which used a 2:1 binding model).

### Results

To rank the clones based on binding affinity, an expression-normalized capture (ENC) assay was performed (Data not shown). The five scFvs displaying the highest binding signals (TPL0039_05_B12, TPL0039_05_F04, TPL0039_05_G08, TPL0039_05_B04, and TPL0039_05_A03) were selected for reformatting to the IgG format, expressed, purified, and their binding to *B*. *asper* myotoxin II was confirmed for all five IgGs through ELISA (Fig. 2A). Following confirmation of retained binding ability, the functional affinity (avidity) of the five IgGs were measured using octet, yielding functional affinities ranging from 9 nM to <1 pM (no dissociation observed) as seen in Fig. 2B.

### Conclusions

The five scFV clones displaying the highest binding signals could be successfully reformatted to the IgG format and purified. The IgG formats displayed great functional affinities ranging from 9 nM to <1 pM.

### Example 3: Investigating neutralization of cytotoxic effects of B. asper myotoxin II using a fibroblast-based assay

### Material and methods

### Human fibroblast assay

A functional in vitro neutralization assay was carried out using human dermal fibroblasts (neonatal; 106-05N, Sigma Aldrich) based on the CellTiter-Glo^{®} Luminescent Cell Viability Assay (Cat.# G7571, Promega, USA) Firstly, the IC50 of *B*. asper myotoxin II was determined in fibroblasts (72.5 µg/mL) and subsequently all IgGs were assessed for their neutralizing abilities. The level of neutralization was tested at toxin:IgG molar ratios of 1:0.5, 1:1, and 1:1.5.

The assay was carried out according to the manufacturer's protocol over a span of three consecutive days and all experiments were run in triplicates: On the first day, 100 µL of cells (400,000 cells/ mL in fibroblast culture medium) were seeded in 96-well plates and grown for 24h at 37°C/ 5% CO2. After 24 hours of incubation, the toxin, or toxin-lgGs mixtures (preincubated 30 minutes at 37°C) were added to their respective wells. Following 24 hours of incubation (37°C/ 5% CO2), the wells were emptied and 100 uL CellTiter-Glo^{®} Reagent (room temperature) was added to each well and the plates were incubated 5-10 minutes on an orbital shaker. After 10 minutes at room temperature the luminescence was recorded (Victor Nivo, Perkin Elmer).

### Results

To test the functional neutralization of the cytotoxic effects of B. *asper* myotoxin II by the five IgGs in vitro, a fibroblast viability assay was carried out (Fig. 3). In this assay, four molar ratios of toxin:IgG were tested; 1:0.5, 1:1, 1:1.5, and 0:1.5. The included controls were: a negative control (PBS), a positive control (toxin challenge without IgG), and a non-myotoxin II binding IgG at toxin:IgG ratios of 1:3 and 0:3. The negative control showed no influence on cell viability, whereas the positive control resulted in -80% reduction in viability. The irrelevant IgG displayed no significant change in viability when compared to the positive control, suggesting that any neutralization observed in the assay for other IgGs is caused by the specificity to myotoxin II.

The in vitro neutralization of the five IgGs can be divided into three patterns. The neutralization of the IgG TPL0039_05_F04 was at all concentrations significantly lower than the negative control, and furthermore resulted in decreased cell viability with increasing IgG amounts. For TPL0039_05_G08 and TPL0039_05_B04, the neutralization capacity was observed to be slightly dose dependent, with increasing IgG ratios leading to an increase in cell viability. TPL0039_5_G08 showed full neutralization (non-significant change from negative control) at 1:1 toxin:IgG ratio. Finally, TPL0039_05_B12, TPL0039_05_B04, and TPL0039_05_A03, displayed full neutralization at the lowest tested 1:0.5 toxin:IgG ratio, and showed no dose response increase with increasing IgG ratios.

### Conclusions

All five IgGs provided neutralization of the toxin in vitro, with full neutralization observed at very low toxin:IgG ratio, down to 1:0.5 toxin:IgG ratio for 2 of the IgG tested.

### Example 4: IgG provides full in vivo neutralization of B. asper myotoxin II toxicity at low dose

### Material and methods

### In vivo mouse assay for myotoxicity neutralization

Myotoxin II from B. asperwas preincubated for 30 min at room temperature with either (a) phosphate-buffered saline (0.12 M NaCl, 0.04 M sodium phosphate; PBS, pH 7.2), (b) monoclonal antibody (TPL0039_05_B12and TPL0039_05_G08) at 1:1 molar ratio, or (c) ICP polyvalent equine antivenom (batch 5991117, Instituto Clodomiro Picado) at 1.6 mg toxin/mL antivenom ratio. Subsequently the preincubated mixtures were injected by intramuscular (gastrocnemius) route, in a total volume of 100 µL (containing 75 µg of toxin as challenge dose), in groups of five CD-1 mice. As a control, a group of mice received an identical injection of 100 µL of PBS alone. Monoclonal antibody alone, in equal amount as in the myotoxin-preincubated mixture, was injected in a group of 5 mice as an additional control. All mice were bled 3 h after injection and the plasma creatine kinase (CK) activity was determined using a UV-kinetic commercial assay (CK-Nac, Biocon Diagnostik), as an indicator of skeletal muscle necrosis (Gutiérrez et al., 1986).

Using the same mouse assay, it was evaluated whether monoclonal antibody TPL0039_05_B12 (153 ug), polyvalent antivenom (29 µL), or polyvalent antivenom (29 uL) mixed with monoclonal antibody TPL0039_05_B12 (125 ug), could neutralize the myotoxic effects of whole B. asper venom. Venom and antibodies were preincubated for 30 min at room temperature, and then the mixtures were intramuscularly injected in groups of 5 mice (100 µL, containing 50 µg of whole venom as challenge dose), using as a control group of mice receiving PBS alone. Plasma CK activity after 3 h was determined as above.

Mouse experiments followed ethical guidelines of the Institutional Committee for the Use and Care of Animals (CICUA, #084-17) of the University of Costa Rica. Statistical significance of the difference between each group and the positive control (Venom) was determined by one-way ANOVA followed by Dunnet's multiple comparisons test using Graphpad Prism version 9.2.0 for macOS.

### Results

From the shown data obtained for the five IgGs, TPL0039_05_B12and TPL0039_05_G08 were evaluated to be the most promising and were selected for investigation of in vivo neutralization of *B*. *asper* myotoxin II (Fig. 4). Mice were injected intramuscularly with preincubated mixtures of *B*. *asper* myotoxin II and IgG in a 1:1 molar ratio. Control mice were injected with either PBS, *B. asper* myotoxin II, *B. asper* myotoxin II and ICP polyvalent equine antivenom, or *B*. *asper* myotoxin II and a non-myotoxin II binding IgG. After three hours, the mice were bled, and the plasma creatine kinase (CK) levels were measured as an indicator of muscle damage. Injection of *B*. *asper* myotoxin II caused a large increase in CK activity levels compared to the PBS-injected control group. Injection of the IgGs without *B*. *asper* myotoxin II resulted in slight increasing trends in the CK levels, although without any statistical increase. When tested, the currently used antivenom for *B*. *asper* bites, ICP polyvalent equine antivenom, showed neutralizing trends, although the CK level increase was still significantly larger compared to the levels observed for the negative control. When testing the two monoclonal IgGs, it was revealed that the IgG TPL0039_05_G08 was unable to prevent the CK increment caused by *B. asper* myotoxin II at a 1:1 toxin:IgG ratio, indicating that this antibody might bind a non-neutralizing epitope. However, the TPL0039_05_B12 IgG IgG was able to fully neutralize the CK level increase caused by *B. asper* myotoxin II (toxin and IgG injection was not significantly higher than the negative controls) at a 1:1 toxin: IgG molar ratio.

### Conclusions

One of the IgG was able to fully neutralize *B*. *asper* myotoxin II toxicity as measured by creatine kinase levels increase neutralization, at low dose of 1:1 toxin:IgG molar ratio.

### Example 5: A combination of TPL0039_05_B12 monoclonal IgG and ICP polyvalent equine antivenom neutralizes muscle damage caused by B. asper whole venom

### Material and methods

The materials and methods used in this example are as described previously herein unless otherwise indicated.

### Results

Lastly, the inventors wanted to assess whether our discovered human IgGs could be used in combination with the ICP polyvalent equine antivenom in vivo. To assess this, the inventors tested if the ICP antivenom supplemented with TPL0039_05_B12 IgG could neutralize the muscle damage caused by B. asper whole venom. In this experiment, mice were injected intramuscularly with preincubated mixtures of B. *asper* whole venom and ICP antivenom supplemented with TPL0039_05_B12 IgG. Control mice were injected with either PBS, B. *asper* venom, B. asper venom and ICP polyvalent equine antivenom, or B. aspervenom and TPL0039_05_B12 IgG. Injection of myotoxin II from B. asper caused a large increase in CK activity levels compared to the PBS-injected control group (Fig. 5). Furthermore, the venom preincubated with TPL0039_05_B12 and venom preincubated with ICP antivenom controls showed partial neutralization of the increased CK levels. When tested together against B. asper venom, ICP antivenom and TPL0039_05_B12 IgG showed CK levels almost identical to the PBS only control, corresponding to full neutralization of the muscle damaging effects caused by the whole venom.

### Conclusions

TPL0039_05_B12 in combination with existing ICP polyvalent equine antivenom show an additive effect in neutralizing muscle damaging effects caused by *B. asper* whole venom as measured by creatine kinase levels.

### Sequence overview

**SEQ. ID. NO:1 :**
   Polypeptide sequence of Myotoxin II (Basic phospholipase A2 homolog 2, uniprot ID P24605) 
SLFELGKMILQETGKNPAKSYGAYGCNCGVLGRGKPKDATDRCCYVHKCCYKKLTG
CNPKKDRYSYSWKDKTIVCGENNSCLKELCECDKAVAICLRENLNTYNKKYRYYLKPL
CKKADAC
**SEQ. ID. NO:2 :**
   Polypeptide sequence of variable heavy chain CDR1 (CDR-H1) of antibodies TPL0039_05_B12, TPL0039_05_F04, TPL0039_05_G08, TPL0039_05_B04, TPL0039_05_A03.
   GFTFSSYG
**SEQ. ID. NO:3 :**
   Polypeptide sequence of variable heavy chain CDR2 (CDR-H2) of antibodies TPL0039_05_B12, TPL0039_05_F04, TPL0039_05_G08, TPL0039_05_B04, TPL0039_05_A03.
   ISYDGSNK
**SEQ. ID. NO:4 :**
   Polypeptide sequence of variable heavy chain CDR3 (CDR-H3) of antibody TPL0039_05_B12
   AGWTEGDAFDI
**SEQ. ID. NO:5 :**
   Polypeptide sequence of variable light chain CDR1 (CDR-L1) of antibodies TPL0039_05_B12, TPL0039_05_F04, TPL0039_05_G08, TPL0039_05_A03. SGSIASNY
**SEQ. ID. NO:6 :**
   Polypeptide sequence of variable light chain CDR2 (CDR-L2) of antibody TPL0039_05_B12, TPL0039_05_G08, TPL0039_05_B04
   EDN
**SEQ. ID. NO:7 :**
   Polypeptide sequence of variable light chain CDR3 (CDR-L3) of antibody TPL0039_05_B12, TPL0039_05_A03.
   QSYDSSSVV
**SEQ. ID. NO:8 :**
   Polypeptide sequence of variable heavy chain CDR3 (CDR-H3) of antibody TPL0039_05_F04
   AKDLEYSSSS
**SEQ. ID. NO:9 :**
   Polypeptide sequence of variable light chain CDR2 (CDR-L2) of antibody TPL0039_05_F04, TPL0039_05_A03.
   EDD
**SEQ. ID. NO:10:**
   Polypeptide sequence of variable light chain CDR3 (CDR-L3) of antibodies TPL0039_05_F04, TPL0039_05_G08, TPL0039_05_B04
   QSYDSSTVV
**SEQ. ID. NO:11 :**
   Polypeptide sequence of variable heavy chain CDR3 (CDR-H3) of antibody TPL0039_05_G08
   ARTNYDSGDAFDI
**SEQ. ID. NO:12 :**
   Polypeptide sequence of variable heavy chain CDR3 (CDR-H3) of antibody TPL0039_05_B04
   ARVGENEAFDI
**SEQ. ID. NO:13:**
   Polypeptide sequence of variable light chain CDR1 (CDR-L1) of antibody TPL0039_05_B04
   SGSIARNY
**SEQ. ID. NO: 14 :**
   Polypeptide sequence of variable heavy chain of antibody TPL0039_05_B12 
EVQLVESGGGVVQPGRSLRLSCAASGFTFSSYGMHWVRQAPGKGLEWVAVISYDG
SNKYYADSVKGRFTISRDNSKNTLYLQMNSLRAEDTAVYYCAGWTEGDAFDIWGQG
TLVTVSS
**SEQ. ID. NO: 15:**
   Polypeptide sequence of variable light chain of antibody TPL0039_05_B12 
NFMLTQPHSVSESPGKTVTISCTRSSGSIASNYVQWYQQRPGSAPSTVIYEDNRRPS
GVPDRFSGSIDSASNSASLTISGLKTEDEADYYCQSYDSSSVVFGGGTKITVLGQP
**SEQ. ID. NO: 16 :**
   Polypeptide sequence of variable heavy chain of antibody TPL0039_05_F04 
QVQLVESGGGVVQPGRSPRLSCAASGFTFSSYGMHWVRQAPGKGLEWVAVISYDG
SNKYYADSVKGRFTISRDNSKNTLYLQMNSLRAEDTAVYYCAKDLEYSSSSWGQGTL
VTVSS
**SEQ. ID. NO: 17:**
   Polypeptide sequence of variable light chain of antibody TPL0039_05_F04 
NFMLTQPHSVSESPGKTVTISCTRSSGSIASNYVQWFQQRPGSSPTTVIYEDDQRPS
GVPDRFSGSIDSSSNSASLTISGLKTEDEATYFCQSYDSSTVVFGGGTKVTVLGQP
**SEQ. ID. NO: 18 :**
   Polypeptide sequence of variable heavy chain of antibody TPL0039_05_G08 
EVQLVESGGGVVQPGRSLRLSCAASGFTFSSYGMHWVRQAPGKGLEWVAVISYDG
SNKYYADSVKGRFTISRDNSKNTLYLQMNSLRAEDTAVYYCARTNYDSGDAFDIWGQ
GTLVTVSS
**SEQ. ID. NO: 19:**
   Polypeptide sequence of variable light chain of antibody TPL0039_05_G08 
NFMLTQPHSVSESPGKTVTISCTRSSGSIASNYVQWYQQRPGSSPTTVIYEDNQRPS
GVPDRFSGSIDSSSNSASLTISGLKTEDEADYYCQSYDSSTVVFGGGTKLTVLGQP
**SEQ. ID. NO: 20:**
   Polypeptide sequence of variable heavy chain of antibody TPL0039_05_B04 
EVQLLESGGGVVQPGRSLRLSCAASGFTFSSYGMHWVRQAPGKGLEWVAVISYDGS
NKYYADSVKGRFTISRDNSKNTLYLQMNSLRAEDTAVYYCARVGENEAFDIWGQGTM
VTVSS
**SEQ. ID. NO: 21:**
   Polypeptide sequence of variable light chain of antibody TPL0039_05_B04 
NFMLTQPHSVSESPGKTVTISCTRSSGSIARNYVQWYQQRPGSAPTTVIYEDNQRPS
GVPDRFSGSIDSSSNSASLTISGLKTEDEADYYCQSYDSSTVVFGGGTKLTVLGQP
**SEQ. ID. NO: 22 :**
   Polypeptide sequence of the B. *asper* Myotoxin II epitope
   KKYRYYLKPLCKK
**SEQ. ID. NO: 23 :**
   Polypeptide sequence of variable heavy chain CDR3 (CDR-H3) of antibody TPL0039_05_A03.
   ASTLQQLDAFDI
**SEQ. ID. NO: 24 :**
   Polypeptide sequence of variable heavy chain of antibody TPL0039_05_A03. 
QVQLVESGGGLAKPGRSLRLSCAVSGFTFSSYGMHWVRQAPGKGLEWVAVISYDGS
NKYYADSVKGRFTISRDNSKNTLYLQMNSLRAEDTAVYYCASTLQQLDAFDIWGQGT
MVTVSS
**SEQ. ID. NO: 25 :**
   Polypeptide sequence of variable light chain of antibody TPL0039 05 A03. 
NFMLTQPHSVSESPGKTITISCTRSSGSIASNYVQWYQQRPGSVPTPVIYEDDRRPSG
VPDRFSGSIDSSSNSASLTISGLKTEDEADYYCQSYDSSSVVFGGGTKITVLGQP
**SEQ. ID. NO: 26 :**
   Polynucleotide sequence of the S10b primer
   GGCTTTGTTAGCAGCCGGATCTCA
**SEQ. ID. NO: 27 :**
   Polynucleotide sequence of the pINT VH Seq Fwd primer
   TGGAGCTGTATCATCCTCTTCTTGG
**SEQ. ID. NO: 28 :**
   Polynucleotide sequence of the pINT VL lambda seq R primer
   ACTCCGGCTTTCACTGGGGAGCTGTCTGCCTTC

### References

W. F. D. Acqua, R. M. Woods, E. S. Ward, S. R. Palaszynski, N. K. Patel, Y. A. Brewah, H. Wu, P. A. Kiener, S. Langermann, Increasing the Affinity of a Human IgG1 for the Neonatal Fc Receptor: Biological Consequences. The Journal of Immunology 169, 5171-5180 (2002).
S. Ahmadi, M. B. Pucca, J. A. Jürgensen, R. Janke, L. Ledsgaard, E. M. Schoof, C. V. Sørensen, F. Çali 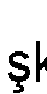 kan, A. H. Laustsen, An in vitro methodology for discovering broadly-neutralizing monoclonal antibodies. Scientific Reports 10, 1-7 (2020).
S. Føns, L. Ledsgaard, M. V. Nikolaev, A. A. Vassilevski, C. V. Sørensen, M. K. Chevalier, M. Fiebig, A. H. Laustsen, Discovery of a Recombinant Human Monoclonal Immunoglobulin G Antibody Against a-Latrotoxin From the Mediterranean Black Widow Spider (Latrodectus tredecimguttatus). Front. Immunol. 11: 587825. doi: 10.3389/fimmu (2020).
J. Gutiérrez, B. Lomonte, L. Cerdas, Isolation and partial characterization of a myotoxin from the venom of the snake Bothrops nummifer. Toxicon 24, 885-894 (1986).
A. H. Laustsen, B. Lohse, B. Lomonte, M. Engmark, J. M. Gutiérrez, Selecting key toxins for focused development of elapid snake antivenoms and inhibitors guided by a Toxicity Score. Toxicon 104, 43-45 (2015).
A. H Laustsen, M. Engmark, C. Milbo, J. Johannesen, B. Lomonte, J. Maria Gutierrez, B. Lohse, From fangs to pharmacology: the future of snakebite envenoming therapy. Current Pharmaceutical Design 22, 5270-5293 (2016).
A. H. Laustsen, A. Karatt-Vellatt, E. W. Masters, A. S. Arias, U. Pus, C. Knudsen, S. Oscoz, P. Slavny, D. T. Griffiths, A. M. Luther, In vivo neutralization of dendrotoxin-mediated neurotoxicity of black mamba venom by oligoclonal human IgG antibodies. Nature communications 9, 3928 (2018).
B. Lomonte, J. Gutiérrez, A new muscle damaging toxin, myotoxin II, from the venom of the snake Bothrops asper (terciopelo). Toxicon 27, 725-733 (1989).
C. D. Martin, G. Rojas, J. N. Mitchell, K. J. Vincent, J. Wu, J. McCafferty, D. J. Schofield, A simple vector system to improve performance and utilisation of recombinant antibodies. BMC biotechnology 6, 46 (2006).
D. Mora-Obando, C. Diaz, Y. Angulo, J. M. Gutiérrez, B. Lomonte, Role of enzymatic activity in muscle damage and cytotoxicity induced by Bothrops asper Asp49 phospholipase A2 myotoxins: are there additional effector mechanisms involved? PeerJ 2, e569 (2014).
D. J. Schofield, A. R. Pope, V. Clementel, J. Buckell, S. D. Chapple, K. F. Clarke, J. S. Conquer, A. M. Crofts, S. R. Crowther, M. R. Dyson, Application of phage display to high throughput antibody generation and characterization. Genome biology 8, 1-18 (2007).
F. W. Studier, Protein production by auto-induction in high-density shaking cultures. Protein expression and purification 41, 207-234 (2005).
D. Xu, M.-L. Alegre, S. S. Varga, A. L. Rothermel, A. M. Collins, V. L. Pulito, L. S. Hanna, K. P. Dolan, P. W. H. I. Parren, J. A. Bluestone, L. K. Jolliffe, R. A. Zivin, In Vitro Characterization of Five Humanized OKT3 Effector Function Variant Antibodies. Cellular Immunology 200, 16-26 (2000).

### Items

The invention may further be defined by any one of the followings items:
1. An antibody capable of neutralizing myotoxin II of SEQ. ID. NO: 1 or a toxin sharing at least 60%, preferably at least 70%, yet more preferably at least 80%, such as at least 90%, for example at least 95%, such as at least 98% sequence identity therewith.
2. The antibody according to item 1, wherein the toxin is a toxin from the venom of a snake from a genus selected from the group consisting of : *Bothrops, Bothrocophias, Trimeresurus, Agkistrodon, Protobothrops, Bothriechis, Cerrophidion, Gloydius, Crotalus, Deinagkistrodon, Atropoides, Cerrophidion, Lachesis, Ovophis, Echis, Vipera, Daboia.;* preferably wherein the genus is *Bothrops.*
3. The antibody according to item 2, wherein the snake from the *Bothrops* genus is selected from the group consisting of: *Bothrops asper, Bothrops alcatraz, Bothrops alternatus, Bothrops ammodytoides, Bothrops atrox, Bothrops ayerbei, Bothrops barnetti, Bothrops bilineatus, Bothrops brazili, Bothrops caribbaeus, Bothrops chloromelas. Bothrops cotiara, Bothrops diporus, Bothrops erythromelas, Bothrops fonsecai, Bothrops insularis, Bothrops itapetiningae, Bothrops jararaca, Bothrops jararacussu, Bothrops jonathani, Bothrops lanceolatus, Bothrops leucurus, Bothrops lutzi, Bothrops marajoensis, Bothrops marmoratus, Bothrops mattogrossensis, Bothrops medusa, Bothrops monsignifer, Bothrops moojeni, Bothrops muriciensis, Bothrops neuwiedi, Bothrops oligobalius, Bothrops oligolepis, Bothrops osbornei, Bothrops otavioi, Bothrops pauloensis, Bothrops pictus, Bothrops pirajai, Bothrops pubescens, Bothrops pulcher, Bothrops punctatus, Bothrops sanctaecrucis, Bothrops sazimai, Bothrops sonene, Bothrops taeniatus and Bothrops venezuelensis.*
4. The antibody according to item 3 wherein the snake from the *Bothrops* species is *Bothrops asper.*
5. The antibody according to any one of the preceding items, wherein said antibody binds said toxin, for example myotoxin II of SEQ. ID. NO: 1 with a K_{d} of at the most 1 pM, for example at the most 10 pM, such as at the most 25 pM, for example at the most 50 pM, such as at the most 100 pM, for example at the most 1 nM, such as at the most 10 nM, for example at the most 50 nM.
6. An antibody comprising or consisting of a heavy chain variable (VH) region comprising a heavy chain complementarity-determining region 3 (CDR-H3) comprising or consisting of SEQ. ID. NO: 4 , or a variant thereof, wherein in said variant up to 3 amino acids have been altered, for example wherein 2 or 1 amino acid(s) have been altered.
7. The antibody according to any one of items 1 to 5, wherein the antibody comprises the sequences according to item 6.
8. The antibody according to any one of the preceding items comprising or consisting of:
   a) a heavy chain variable (VH) region comprising a heavy chain complementarity-determining region 3 (CDR-H3) comprising or consisting of any of SEQ. ID. NOs: 4, 8, 11, 12, or a variant thereof, wherein in said variant up to 3 amino acids have been altered, for example wherein 2 or 1 amino acid(s) have been altered;
      and
   b) a light chain variable (VL) region comprising a light chain complementarity-determining region 3 (CDR-L3) comprising or consisting of any of SEQ. ID. NOs: 7, 10, or a variant thereof, wherein in said variant up to 3 amino acids have been altered, for example wherein 2 or 1 amino acid(s) have been altered.
9. The antibody according to any one of the preceding items comprising or consisting of:
   a) a heavy chain variable (VH) region comprising:
      i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
      ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and iii. a heavy chain complementarity-determining region 3 (CDR-H3) comprising or consisting of any of SEQ. ID. NO: 4, 8, 11, 12, or a variant thereof, wherein in said variant up to 3 amino acids have been altered, for example wherein 2 or 1 amino acid(s) have been altered;
      and
   b) a light chain variable (VL) region comprising:
      iv. a light chain complementarity-determining region 1 (CDR-L1) comprising or consisting of any of SEQ. ID. NO: 5, 13, or a variant thereof, wherein in said variant up to 3 amino acids have been altered, for example wherein 2 or 1 amino acid(s) have been altered;
      v. a light chain complementarity-determining region 2 (CDR-L2) comprising or consisting of any of SEQ. ID. NO: 6, 9, or a variant thereof, wherein in said variant up to 3 amino acids have been altered, for example wherein 2 or 1 amino acid(s) have been altered; and
      vi. a light chain complementarity-determining region 3 (CDR-L3) comprising or consisting of SEQ. ID. NO: 7, 10, or a variant thereof, wherein in said variant up to 3 amino acids have been altered, for example wherein 2 or 1 amino acid(s) have been altered.
10. An antibody comprising or consisting of:
   a) a heavy chain variable (VH) region comprising:
      i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
      ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
      iii. a heavy chain complementarity-determining region 3 (CDR-H3) comprising or consisting of SEQ. ID. NO: 4 or a variant thereof, wherein in said variant up to 3 amino acids have been altered, for example wherein 2 or 1 amino acid(s) have been altered;
      and
   b) a light chain variable (VL) region comprising:
      vii. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
      viii. a light chain complementarity-determining region 2 (CDR-L2) comprising or consisting of SEQ. ID. NO: 6 or a variant thereof, wherein in said variant up to 3 amino acids have been altered, for example wherein 2 or 1 amino acid(s) have been altered; and
      ix. a light chain complementarity-determining region 3 (CDR-L3) comprising or consisting of SEQ. ID. NO: 7 or a variant thereof, wherein in said variant up to 3 amino acids have been altered, for example wherein 2 or 1 amino acid(s) have been altered.
11. The antibody according to any one of the preceding items, wherein the antibody comprises or consists of:
   a) a heavy chain variable (VH) region comprising:
      i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
      ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
      iii. a heavy chain complementarity-determining region 3 (CDR-H3) comprising or consisting of SEQ. ID. NO: 8 or a variant thereof, wherein in said variant up to 3 amino acids have been altered, for example wherein 2 or 1 amino acid(s) have been altered;
      and
   b) a light chain variable (VL) region comprising:
      iv. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
      v. a light chain complementarity-determining region 2 (CDR-L2) comprising or consisting of SEQ. ID. NO: 9 or a variant thereof, wherein in said variant up to 3 amino acids have been altered, for example wherein 2 or 1 amino acid(s) have been altered; and
      vi. a light chain complementarity-determining region 3 (CDR-L3) comprising or consisting of SEQ. ID. NO: 10 or a variant thereof, wherein in said variant up to 3 amino acids have been altered, for example wherein 2 or 1 amino acid(s) have been altered.
12. The antibody according to any one of the preceding items, wherein the antibody comprises or consists of:
   a) a heavy chain variable (VH) region comprising:
      i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
      ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acids have been altered; and
      iii. a heavy chain complementarity-determining region 3 (CDR-H3) comprising or consisting of SEQ. ID. NO: 11 or a variant thereof, wherein in said variant up to 3 amino acids have been altered, for example wherein 2 or 1 amino acids have been altered;
      and
   b) a light chain variable (VL) region comprising:
      i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
      ii. a light chain complementarity-determining region 2 (CDR-L2) comprising or consisting of SEQ. ID. NO: 6 or a variant thereof, wherein in said variant up to 3 amino acids have been altered, for example wherein 2 or 1 amino acid(s) have been altered; and
      iii. a light chain complementarity-determining region 3 (CDR-L3) comprising or consisting of SEQ. ID. NO: 10 or a variant thereof, wherein in said variant up to 3 amino acids have been altered , for example wherein 2 or 1 amino acid(s) have been altered .
13. The antibody according to any one of the preceding items, wherein the antibody comprises or consists of:
   a) a heavy chain variable (VH) region comprising:
      i. a heavy chain complementarity-determining region 1 (CDR-H1) comprising or consisting of SEQ. ID. NO: 2 or a variant thereof, wherein in said variant up to 3 amino acids have been altered , for example wherein 2 or 1 amino acid(s) have been altered ;
      ii. a heavy chain complementarity-determining region 2 (CDR-H2) comprising or consisting of SEQ. ID. NO: 3 or a variant thereof, wherein in said variant up to 3 amino acids have been altered , for example wherein 2 or 1 amino acid(s) have been altered ; and
      iii. a heavy chain complementarity-determining region 3 (CDR-H3) comprising or consisting of SEQ. ID. NO: 12 or a variant thereof, wherein in said variant up to 3 amino acids have been altered , for example wherein 2 or 1 amino acids have been altered ;
      and
   b) a light chain variable (VL) region comprising:
      iv. a light chain complementarity-determining region 1 (CDR-L1) comprising or consisting of SEQ. ID. NO: 13 or a variant thereof, wherein in said variant up to 3 amino acids have been altered , for example wherein 2 or 1 amino acid(s) have been altered ;
      v. a light chain complementarity-determining region 2 (CDR-L2) comprising or consisting of SEQ. ID. NO: 6 or a variant thereof, wherein in said variant up to 3 amino acids have been altered , for example wherein 2 or 1 amino acid(s) have been altered ; and
      vi. a light chain complementarity-determining region 3 (CDR-L3) comprising or consisting of SEQ. ID. NO: 10 or a variant thereof, wherein in said variant up to 3 amino acids have been altered , for example wherein 2 or 1 amino acid(s) have been altered .
14. The antibody according to any one of the preceding items, wherein the antibody comprises or consists of:
   a) a heavy chain variable (VH) region comprising:
      i. a heavy chain complementarity-determining region 1 (CDR-H1) comprising or consisting of SEQ. ID. NO: 2 or a variant thereof, wherein in said variant up to 3 amino acids have been altered , for example wherein 2 or 1 amino acid(s) have been altered ;
      ii. a heavy chain complementarity-determining region 2 (CDR-H2) comprising or consisting of SEQ. ID. NO: 3 or a variant thereof, wherein in said variant up to 3 amino acids have been altered , for example wherein 2 or 1 amino acid(s) have been altered ; and
      iii. a heavy chain complementarity-determining region 3 (CDR-H3) comprising or consisting of SEQ. ID. NO: 23 or a variant thereof, wherein in said variant up to 3 amino acids have been altered , for example wherein 2 or 1 amino acid(s) have been altered ;
      and
   b) a light chain variable (VL) region comprising:
      i. a light chain complementarity-determining region 1 (CDR-L1) comprising or consisting of SEQ. ID. NO: 5 or a variant thereof, wherein in said variant up to 3 amino acids have been altered , for example wherein 2 or 1 amino acid(s) have been altered ;
      ii. a light chain complementarity-determining region 2 (CDR-L2) comprising or consisting of SEQ. ID. NO: 9; and
      iii. a light chain complementarity-determining region 3 (CDR-L3) comprising or consisting of SEQ. ID. NO: 7 or a variant thereof, wherein in said variant up to 3 amino acids have been altered , for example wherein 2 or 1 amino acid(s) have been altered .
15. The antibody according to any one of the preceding items, wherein the protein comprises or consists of:
   a) a heavy chain variable (VH) region comprising or consisting of any of SEQ. ID. NO: 14, 16, 18, 20 or a variant thereof, wherein in said variant up to 5 amino acids have been altered, for example wherein 4, 3, 2, or 1 amino acid(s) have been altered; and
   b) a light chain variable (VL) region comprising or consisting of any of SEQ. ID. NO: 15, 17, 19, 21 or a variant thereof, wherein in said variant up to 5 amino acids have been altered, for example wherein 4, 3, 2, or 1 amino acid(s) have been altered.
16. The antibody according to any one of the preceding items, wherein the protein comprises or consists of:
   a) a heavy chain variable (VH) region comprising or consisting of SEQ. ID. NO: 14, or a variant thereof, wherein in said variant up to 5 amino acids have been altered , for example wherein 4, 3, 2, or 1 amino acid(s) have been altered ; and
   b) a light chain variable (VL) region comprising or consisting of SEQ. ID. NO: 15, or a variant thereof, wherein in said variant up to 5 amino acids have been altered, for example wherein 4, 3, 2, or 1 amino acid(s) have been altered.
17. The antibody according to any one of the preceding items, wherein the protein comprises or consists of:
   a) a heavy chain variable (VH) region comprising or consisting of SEQ. ID. NO: 16, or a variant thereof, wherein in said variant up to 5 amino acids have been altered , for example wherein 4, 3, 2, or 1 amino acid(s) have been altered ; and
   b) a light chain variable (VL) region comprising or consisting of SEQ. ID. NO: 17, or a variant thereof, wherein in said variant up to 5 amino acids have been altered , for example wherein 4, 3, 2, or 1 amino acid(s) have been altered.
18. The antibody according to any one of the preceding items, wherein the protein comprises or consists of:
   a) a heavy chain variable (VH) region comprising or consisting of SEQ. ID. NO: 18, or a variant thereof, wherein in said variant up to 5 amino acids have been altered , for example wherein 4, 3, 2, or 1 amino acid(s) have been altered ; and
   c) a light chain variable (VL) region comprising or consisting of SEQ. ID. NO: 19, or a variant thereof, wherein in said variant up to 5 amino acids have been altered , for example wherein 4, 3, 2, or 1 amino acid(s) have been altered.
19. The antibody according to any one of the preceding items, wherein the protein comprises or consists of:
   a) a heavy chain variable (VH) region comprising or consisting of SEQ. ID. NO: 20, or a variant thereof, wherein in said variant up to 5 amino acids have been altered , for example wherein 4, 3, 2, or 1 amino acid(s) have been altered ; and
   c) a light chain variable (VL) region comprising or consisting of SEQ. ID. NO: : 21, or a variant thereof, wherein in said variant up to 5 amino acids have been altered , for example wherein 4, 3, 2, or 1 amino acid(s) have been altered.
20. The antibody according to any one of the preceding items, wherein the protein comprises or consists of:
   a) a heavy chain variable (VH) region comprising or consisting of SEQ. ID. NO: 24; and
   b) a light chain variable (VL) region comprising or consisting of SEQ. ID. NO: 25.
21. The antibody according to any one of items 6 to 20, wherein the one or more amino acids that have been altered is (are) an amino acid(s) not part of the CDR as defined in any one of items 6 to 14.
22. The antibody according to any one of items 6 to 21, wherein the one or more amino acids that have been altered, have been substituted by another amino acid.
23. The antibody according to any one of the preceding items, wherein the VL and/or the VH region further comprises an N-terminal signal peptide sequence.
24. The antibody according to any one of the preceding items, wherein the antibody is selected from the group consisting of a full-length antibody, a Fab fragment, a F(ab') fragment, a F(ab')₂ fragment, an scFv, a diabody, and a triabody.
25. The antibody according to any one of the preceding items comprising an immunoglobulin constant region.
26. The antibody according to any one of the preceding items comprising an immunoglobulin constant region selected from the group consisting of IgG, IgM, IgA, IgD, and IgE, preferably selected from the group consisting of human IgG, human IgM, human IgA, human IgD, and human IgE.
27. The antibody according to any one of the preceding items comprising an immunoglobulin constant region selected from IgG or IgA.
28. The antibody according to any one of the preceding items comprising an immunoglobulin constant region selected from the group consisting of IgG1, IgA1, and IgA2.
29. The antibody according to any one of the preceding items comprising an immunoglobulin constant region selected from IgG1, IgG2, IgG3, and IgG4.
30. The antibody according to any one of the preceding items, wherein the protein is a monoclonal antibody.
31. The antibody according to any one of the preceding items, wherein the protein comprises a human variable region.
32. The antibody according to any one of the preceding items, wherein the protein is a human antibody or a chimeric antibody.
33. The antibody according to any one of the preceding items, wherein the protein has one or more modification(s) selected from the group consisting of PEGylation, polysialylation, Fc region mutation and N-glycosylation.
34. The antibody according to any one of the preceding items, wherein the protein comprises a detection label.
35. The antibody according to item 34, wherein the detection label is selected from the group consisting of a colorimetric, a fluorescent, a luminescent, a magnetic, and a paramagnetic label.
36. The antibody according to any one of items 34 to 35, wherein the detection label is biotin.
37. The antibody according to any one of items 34 to 36, wherein the detection label is a gold nanoparticle
38. The antibody according to any one of the preceding items wherein the antibody is capable of neutralizing said toxin as determined by a method comprising the steps of:
   - mixing said toxin with said antibody at a molar ratio of no more than 1:5, for instance no more than 1:4, such as no more than 1:3, for instance no more than 1:2, such as no more than 1:1.5, for instance no more than 1:1, such as no more than 1:0.5
   - incubating human cells with said mixture under conditions allowing growth of the cells,
   - determining viability of said cells
   wherein a viability of at least 70%, such as at least 80%, for example at least 90% compared to cells incubated under the same conditions in the absence of said toxin and said antibody is indicative of said antibody neutralising said toxin.
39. The antibody according to any one of the preceding items wherein the antibody is capable of neutralizing said toxin as determined by a method comprising the steps of:
   - mixing said toxin with said antibody at a molar ratio of no more than 1:5, for instance no more than 1:4, such as no more than 1:3, for instance no more than 1:2, such as no more than 1:1.5, for instance no more than 1:1,
   - administering said mixture to one or more mice,
   - determining the plasma creatine kinase (CK) activity in said mice,
   wherein a level of CK activity of less than 50%, preferably less than 40%, such as less than 30%, for example less than 20%, such as less than 10% compared to the CK activity in mice having received the same dosage of said toxin, but no antibody is indicative of said antibody neutralising said toxin.
40. The antibody according to any one of the preceding items, wherein the antibody is capable to bind to an epitope contained between residues corresponding to amino acids 105 to 117 of the myotoxin II protein sequence as defined in SEQ. ID. No 1 or variant(s) thereof having at least 70% identity, such as at least 75% identity, for example at least 80% identity, such as at least 85% identity, for example at least 90% identity, such as at least 95% identity thereto.
41. A pharmaceutical composition comprising an antibody as defined in any one of the preceding items, and a pharmaceutically acceptable diluent, carrier and/or excipient.
42. The pharmaceutical composition of item 41, further comprising one or more additional antigen-binding protein(s) capable of binding to, blocking, or neutralizing one or more snake venom antigen(s).
43. The pharmaceutical composition of item 42, wherein the additional antigen-binding protein(s) capable of binding to, blocking, or neutralizing one or more snake venom antigen(s) is one or more polyclonal antibodies.
44. The pharmaceutical composition of item 42, wherein the additional antigen-binding protein(s) capable of binding to, blocking, or neutralizing one or more snake venom antigen(s) is a snake antivenom.
45. The pharmaceutical composition of item 41, further comprising an active agent suitable for the treatment of snake envenomation.
46. A kit-of-parts comprising
   a) an antibody according to anyone of items 1 to 40; and
   b) an active agent suitable for the treatment of snake envenomation.
47. The composition or the kit-of parts according to any one of items 45 to 46, wherein said active agent is one or more antigen-binding protein(s) capable of binding to, blocking, and/or neutralizing one or more snake venom antigen(s)
48. The composition or the kit-of parts according to any one of items 45 to 46, wherein said other agent used for the treatment of snake envenomation is one or more antigen-binding protein(s) selected from the group consisting of: IgG, IgM, scFv, F(ab)₂, Fab, V_{H}H antibody(ies), and fragments thereof.
49. The composition or the kit-of parts according to any one of items 45 to 48, wherein the one or more antigen-binding protein(s) capable of neutralizing one or more snake venom antigen(s) is (are) selected from the group consisting of: Equine immunoglobulin(s) against snake venom, such as Equine IgG(s) or Equine Fab or Equine F(ab')₂fragment(s) against snake venom, Ovine immunoglobulin(s) against snake venom, such as Ovine IgG(s) or Ovine Fab or Ovine F(ab')₂fragment(s) against snake venom, Rabbit immunoglobulin(s) against snake venom, such as Rabbit IgG(s) or Rabbit Fab or Rabbits F(ab')₂ fragment(s) against snake venom, and Camelids immunoglobulin(s) against snake venom, such as Camelids IgG(s) or Camelids Fab or Camelids F(ab')₂ fragment(s) against snake venom.
50. The composition or the kit-of parts according to any one of items 45 to 49, wherein said other agent used for the treatment of snake envenomation is one or more protein(s) displaying neutralization potential against snake venoms.
51. The composition or the kit-of-parts according to item 50, wherein the one or more protein(s) displaying neutralization potential against snake venoms is (are) selected from the group consisting of: a PLA₂ inhibitor, a matrix metalloproteinase (MMP) inhibitor, a snake venom metalloproteinase inhibitor (SVMP), a Kunitz-type trypsin inhibitor, an antimyotoxic factor, an antimyonecrotic factor, an antineurotoxic factor, an antihæmmoragic factor, an antioedematogenic factor and a glycoprotein, such as acid-a1-glycoprotein.
52. The composition or kit-of-parts according to any one of items 50 to 51, wherein said other agent used for the treatment of snake envenomation is one or more small molecule(s) with inhibitory effect against snake toxins.
53. The composition or kit-of-parts according to item 52, wherein the one or more small molecule(s) with inhibitory effect against snake toxins is (are) selected from the group consisting of: polyanions, polyphenolic compounds, and metalloproteinase inhibitors.
54. The composition or kit-of-parts according to any one of items 52 and 53, wherein the one or more small molecule(s) with inhibitory effect against snake toxins is (are) selected from the list consisting of: Suramin, Heparin, Wedelolcatone, Coumestrol, Glycyrrhizin, Fucoidan, Apigenin analogue, AG-3340, Aristolochic acid, Rosmarinic acid, Imidazopyridine, BAPTA, TPEN, EDTA, DMPS, dimercaprol, BAY-129566, Marimastat, CGS-27023A, Batimastat, Nafamostat, MV 8612 from Mandevilla velutina, MV 8608 from Mandevilla velutina, Koninginin, 4-nerolidylcatechol, Anisodamine, 12-methoxy-4-methylvoachalotine (MMV), Quinonoid xanthene ehretianone, Benzoylsalireposide, Salireposide, Chlorogenic acid, Caffeic acid, 2-hydroxy-4-methoxy benzoic acid and, Varespladib.
55. An antibody according to any one of items 1 to 40, a pharmaceutical composition according to any one of items 41 to 54 or a kit-of-parts according to item 44 to 54 for use in a method of treatment of snake envenomation in a subject.
56. The antibody, the pharmaceutical composition or the kit-of-parts for the use according to item 55, wherein the antibody, the pharmaceutical composition or the kit-of-parts prevents or delay venom-induced lethality.
57. The antibody, the pharmaceutical composition or the kit-of-parts for the use according to any one of items 55 to 56, wherein the antibody, the pharmaceutical composition or the kit-of-parts prevents or delays envenomation symptoms.
58. The antibody, the pharmaceutical composition or the kit-of-parts for the use according to item 57, wherein the envenomation symptoms comprise pain, oozing from the puncture wounds, local swelling, bruising, blisters, numbness, mild fever, headache, tissue damage, muscle damage, kidney injury, dermonecrosis, bleeding from the nose and gums, hemoptysis, gastrointestinal bleeding, hematuria, hypotension, nausea, vomiting, impaired consciousness and tenderness of the spleen.
59. The antibody, the pharmaceutical composition or the kit-of-parts for the use according to any one of items 55 to 58, wherein the antibody, the pharmaceutical composition or the kit-of-parts prevents or delays necrosis and/or gangrene.
60. The antibody, the pharmaceutical composition or the kit-of-parts for the use according to any one of items 55 to 59, wherein the antibody, the pharmaceutical composition or the kit-of-parts prevents the increase or reduces myonecrosis as determined for example by the level of creatine kinase in the subject following the envenomation.
61. The antibody, the pharmaceutical composition or the kit-of-parts for the use according to any one of items 55 to 60, wherein the antibody, the pharmaceutical composition or the kit-of-parts is administered parenterally or pulmonarily.
62. The antibody, the pharmaceutical composition or the kit-of-parts for the use according to item 61, wherein the parenteral administration of the antibody, the pharmaceutical composition or the kit-of-parts is intravenous, intramuscular and/or sub-cutaneous.
63. The antibody, the pharmaceutical composition or the kit-of-parts for the use according to any one of items 55 to 62, wherein the antibody, the pharmaceutical composition or the kit-of-parts is administered no more than 60 min after envenomation, such as no more than 6h after envenomation, for instance no more than 12h after envenomation, such as no more than 24h after envenomation, for instance no more than 48h after envenomation, such as no more than 72h after envenomation, for instance no more than 96h after envenomation, such as no more than 120h after envenomation for instance no more than 144h after envenomation.
64. A method of treating snake envenomation which comprises administering to a subject in need thereof an effective amount of the antibody according to any one of items 1 to 40, the pharmaceutical composition according to any one of items 41 to 54 or the kit-of-parts according to any one of items 44 to 54.
65. Use of the antibody according to any one of items 1 to 40, the composition according to any one of items 41 to 54 or the kit-of-parts according to item 44 to 54 for the manufacture of a medicament for the treatment of snake envenomation.
66. A kit for detection and/or quantification of snake venom in a sample comprising:
   - one or more antibodies, such as two antibodies, wherein at least one of said antibodies is according to any one of items 1 to 40; and
   - means for detection of a complex comprising said one or more antibodies bound to snake venom.
67. The kit according to item 66, wherein the kit further comprises means for acquiring or isolating a sample from a subject.
68. The kit according to any one of items 66 to 67, wherein the sample is a blood sample, a urine sample, a smear of the bite site or a smear of the bite wound.
69. The kit according to item 68, wherein the blood sample is a plasma blood sample.
70. The kit according to any one items 66 to 69, wherein the kit is an immunoassay, such as a lateral flow assay, an ELISA, an electrochemical impedance spectroscopy-based assay, a Bilayer Interferometry (BLI)-based assay or a turbidimetric immunoassay.
71. An antibody according to any one of items 1 to 40 for use in the diagnosis of snake envenomation in a subject.
72. Use of an antibody according to any one of items 1 to 40 in an *in vitro* method for detection and/or diagnosis of snake envenomation in a subject.
73. The antibody, the pharmaceutical composition or the kit-of-parts for the use according to any one of items 55 to 63, the kit according to any one of items 66 to 70, or the antibody for use according to item 71 wherein the subject is a human.
74. The antibody, the pharmaceutical composition or the kit-of-parts for the use according to any one of items 55 to 63, the method according to item 64, use according to item 65 and 72, the kit according to any one of items 66 to 70, or the antibody for use according to item 71, wherein the subject is a non-human animal, wherein said non-human animal for example may belong to the family of Equidae, Bovidae, Canidae or Felidae.
75. The antibody, the pharmaceutical composition, the method, use or the kit-of-parts or the kit according to item 74, wherein the non-human animal is a horse, a cow, an ox, a dog or a cat.
76. A method for detecting snake venom in a sample, said method comprising:
   - providing a sample for analysis;
   - contacting the sample with one or more antibodies as defined in any one of items 1 to 40 to said sample; and
   - detecting a complex comprising said one or more antibodies bound to a snake antigen.
77. The method according to item 76, wherein the method is an *in vitro* method.
78. The method according to any one of items 76 to 77, wherein the detection of the complex is performed by an immunoassay, such as a lateral flow assay, an ELISA, an electrochemical impedance spectroscopy-based assay, a Bilayer Interferometry (BLI)-based assay or a turbidimetric immunoassay.

## Claims

1. An antibody capable of neutralizing myotoxin II of SEQ. ID. NO: 1 or a toxin sharing at least 60%, preferably at least 70%, yet more preferably at least 80%, such as at least 90%, for example at least 95%, such as at least 98% sequence identity therewith.

2. The antibody according to claim 1, wherein the toxin is a toxin from the venom of a snake from a genus selected from the group consisting of : *Bothrops, Bothrocophias, Trimeresurus, Agkistrodon, Protobothrops, Bothriechis, Cerrophidion, Gloydius, Crotalus, Deinagkistrodon, Atropoides, Cerrophidion, Lachesis, Ovophis, Echis, Vipera, Daboia,* preferably wherein the snake from the *Bothrops* genus is selected from the group consisting of : *Bothrops asper, Bothrops alcatraz, Bothrops alternatus, Bothrops ammodytoides, Bothrops atrox, Bothrops ayerbei, Bothrops barnetti, Bothrops bilineatus, Bothrops brazili, Bothrops caribbaeus, Bothrops chloromelas. Bothrops cotiara, Bothrops diporus, Bothrops erythromelas, Bothrops fonsecai, Bothrops insularis, Bothrops itapetiningae, Bothrops jararaca, Bothrops jararacussu, Bothrops jonathani, Bothrops lanceolatus, Bothrops leucurus, Bothrops lutzi, Bothrops marajoensis, Bothrops marmoratus, Bothrops mattogrossensis, Bothrops medusa, Bothrops monsignifer, Bothrops moojeni, Bothrops muriciensis, Bothrops neuwiedi, Bothrops oligobalius, Bothrops oligolepis, Bothrops osbornei, Bothrops otavioi, Bothrops pauloensis, Bothrops pictus, Bothrops pirajai, Bothrops pubescens, Bothrops pulcher, Bothrops punctatus, Bothrops sanctaecrucis, Bothrops sazimai, Bothrops sonene, Bothrops taeniatus and Bothrops venezuelensis.*

3. The antibody according to claim 2 wherein the snake from the *Bothrops* species is *Bothrops asper.*

4. An antibody comprising or consisting of a heavy chain variable (VH) region comprising a heavy chain complementarity-determining region 3 (CDR-H3) comprising or consisting of SEQ. ID. NO: 4 , or a variant thereof, wherein in said variant up to 3 amino acids have been altered, for example wherein 2 or 1 amino acid(s) have been altered.

5. The antibody according to any one of claims 1 to 3, wherein the antibody comprises the sequences according to claim 4.

6. An antibody according to any one of the preceding claims comprising or consisting of:
a) a heavy chain variable (VH) region comprising a heavy chain complementarity-determining region 3 (CDR-H3) comprising or consisting of any of SEQ. ID. NOs: 4, 8, 11, 12, or a variant thereof, wherein in said variant up to 3 amino acids have been altered, for example wherein 2 or 1 amino acid(s) have been altered;
and
b) a light chain variable (VL) region comprising a light chain complementarity-determining region 3 (CDR-L3) comprising or consisting of any of SEQ. ID. NOs: 7, 10, or a variant thereof, wherein in said variant up to 3 amino acids have been altered, for example wherein 2 or 1 amino acid(s) have been altered; or
a) a heavy chain variable (VH) region comprising:
i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
iii. a heavy chain complementarity-determining region 3 (CDR-H3) comprising or consisting of any of SEQ. ID. NO: 4, 8, 11, 12, or a variant thereof, wherein in said variant up to 3 amino acids have been altered, for example wherein 2 or 1 amino acid(s) have been altered;
and
b) a light chain variable (VL) region comprising:
i. a light chain complementarity-determining region 1 (CDR-L1) comprising or consisting of any of SEQ. ID. NO: 5, 13, or a variant thereof, wherein in said variant up to 3 amino acids have been altered, for example wherein 2 or 1 amino acid(s) have been altered;
ii. a light chain complementarity-determining region 2 (CDR-L2) comprising or consisting of any of SEQ. ID. NO: 6, 9, or a variant thereof, wherein in said variant up to 3 amino acids have been altered, for example wherein 2 or 1 amino acid(s) have been altered; and
iii. a light chain complementarity-determining region 3 (CDR-L3) comprising or consisting of SEQ. ID. NO: 7, 10, or a variant thereof, wherein in said variant up to 3 amino acids have been altered, for example wherein 2 or 1 amino acid(s) have been altered.

7. The antibody according to any one of the preceding claims, wherein said antigen-binding protein comprises or consists of:
a) a heavy chain variable (VH) region comprising:
i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
iii. a heavy chain complementarity-determining region 3 (CDR-H3) comprising or consisting of SEQ. ID. NO: 4 or a variant thereof, wherein in said variant up to 3 amino acids have been altered, for example wherein 2 or 1 amino acid(s) have been altered;
and
b) a light chain variable (VL) region comprising:
i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
ii. a light chain complementarity-determining region 2 (CDR-L2) comprising or consisting of SEQ. ID. NO: 6 or a variant thereof, wherein in said variant up to 3 amino acids have been altered, for example wherein 2 or 1 amino acid(s) have been altered; and
iii. a light chain complementarity-determining region 3 (CDR-L3) comprising or consisting of SEQ. ID. NO: 7 or a variant thereof, wherein in said variant up to 3 amino acids have been altered, for example wherein 2 or 1 amino acid(s) have been altered; or
a) a heavy chain variable (VH) region comprising:
i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acid(s) have been altered; and
iii. a heavy chain complementarity-determining region 3 (CDR-H3) comprising or consisting of SEQ. ID. NO: 8 or a variant thereof, wherein in said variant up to 3 amino acids have been altered, for example wherein 2 or 1 amino acid(s) have been altered;
and
b) a light chain variable (VL) region comprising:
i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
ii. a light chain complementarity-determining region 2 (CDR-L2) comprising or consisting of SEQ. ID. NO: 9 or a variant thereof, wherein in said variant up to 3 amino acids have been altered, for example wherein 2 or 1 amino acid(s) have been altered; and
iii. a light chain complementarity-determining region 3 (CDR-L3) comprising or consisting of SEQ. ID. NO: 10 or a variant thereof, wherein in said variant up to 3 amino acids have been altered, for example wherein 2 or 1 amino acid(s) have been altered;
or
a) a heavy chain variable (VH) region comprising:
i. a heavy chain complementarity-determining region 1 (CDR-H1) comprising or consisting of SEQ. ID. NO: 2 or a variant thereof, wherein in said variant up to 3 amino acids have been altered, for example wherein 2 or 1 amino acid(s) have been altered;
ii. a heavy chain complementarity-determining region 2 (CDR-H2) comprising or consisting of SEQ. ID. NO: 3 or a variant thereof, wherein in said variant up to 3 amino acids have been altered, for example wherein 2 or 1 amino acids have been altered; and
iii. a heavy chain complementarity-determining region 3 (CDR-H3) comprising or consisting of SEQ. ID. NO: 11 or a variant thereof, wherein in said variant up to 3 amino acids have been altered, for example wherein 2 or 1 amino acids have been altered;
and
b) a light chain variable (VL) region comprising:
i. a light chain complementarity-determining region 1 (CDR-L1) comprising or consisting of SEQ. ID. NO: 5 or a variant thereof, wherein in said variant up to 3 amino acids have been altered, for example wherein 2 or 1 amino acid(s) have been altered;
ii. a light chain complementarity-determining region 2 (CDR-L2) comprising or consisting of SEQ. ID. NO: 6 or a variant thereof, wherein in said variant up to 3 amino acids have been altered, for example wherein 2 or 1 amino acid(s) have been altered; and
iii. a light chain complementarity-determining region 3 (CDR-L3) comprising or consisting of SEQ. ID. NO: 10 or a variant thereof, wherein in said variant up to 3 amino acids have been altered , for example wherein 2 or 1 amino acid(s) have been altered;
or
a) a heavy chain variable (VH) region comprising:
i. a heavy chain complementarity-determining region 1 (CDR-H1) comprising or consisting of SEQ. ID. NO: 2 or a variant thereof, wherein in said variant up to 3 amino acids have been altered , for example wherein 2 or 1 amino acid(s) have been altered ;
ii. a heavy chain complementarity-determining region 2 (CDR-H2) comprising or consisting of SEQ. ID. NO: 3 or a variant thereof, wherein in said variant up to 3 amino acids have been altered , for example wherein 2 or 1 amino acid(s) have been altered ; and
iii. a heavy chain complementarity-determining region 3 (CDR-H3) comprising or consisting of SEQ. ID. NO: 12 or a variant thereof, wherein in said variant up to 3 amino acids have been altered , for example wherein 2 or 1 amino acids have been altered ;
and
b) a light chain variable (VL) region comprising:
i. a light chain complementarity-determining region 1 (CDR-L1) comprising or consisting of SEQ. ID. NO: 13 or a variant thereof, wherein in said variant up to 3 amino acids have been altered , for example wherein 2 or 1 amino acid(s) have been altered ;
ii. a light chain complementarity-determining region 2 (CDR-L2) comprising or consisting of SEQ. ID. NO: 6 or a variant thereof, wherein in said variant up to 3 amino acids have been altered , for example wherein 2 or 1 amino acid(s) have been altered ; and
iii. a light chain complementarity-determining region 3 (CDR-L3) comprising or consisting of SEQ. ID. NO: 10 or a variant thereof, wherein in said variant up to 3 amino acids have been altered , for example wherein 2 or 1 amino acid(s) have been altered;
or
a) a heavy chain variable (VH) region comprising:
i. a heavy chain complementarity-determining region 1 (CDR-H1) comprising or consisting of SEQ. ID. NO: 2 or a variant thereof, wherein in said variant up to 3 amino acids have been altered , for example wherein 2 or 1 amino acid(s) have been altered ;
ii. a heavy chain complementarity-determining region 2 (CDR-H2) comprising or consisting of SEQ. ID. NO: 3 or a variant thereof, wherein in said variant up to 3 amino acids have been altered , for example wherein 2 or 1 amino acid(s) have been altered ; and
iii. a heavy chain complementarity-determining region 3 (CDR-H3) comprising or consisting of SEQ. ID. NO: 23 or a variant thereof, wherein in said variant up to 3 amino acids have been altered , for example wherein 2 or 1 amino acid(s) have been altered ;
and
b) a light chain variable (VL) region comprising:
i. a light chain complementarity-determining region 1 (CDR-L1) comprising or consisting of SEQ. ID. NO: 5 or a variant thereof, wherein in said variant up to 3 amino acids have been altered , for example wherein 2 or 1 amino acid(s) have been altered ;
ii. a light chain complementarity-determining region 2 (CDR-L2) comprising or consisting of SEQ. ID. NO: 9 ; and
iii. a light chain complementarity-determining region 3 (CDR-L3) comprising or consisting of SEQ. ID. NO: 7 or a variant thereof, wherein in said variant up to 3 amino acids have been altered , for example wherein 2 or 1 amino acid(s) have been altered; or
a) a heavy chain variable (VH) region comprising or consisting of any of SEQ. ID. NO: 14, 16, 18, 20 or a variant thereof, wherein in said variant up to 5 amino acids have been altered, for example wherein 4, 3, 2, or 1 amino acid(s) have been altered; and
b) a light chain variable (VL) region comprising or consisting of any of SEQ. ID. NO: 15, 17, 19, 21 or a variant thereof, wherein in said variant up to 5 amino acids have been altered, for example wherein 4, 3, 2, or 1 amino acid(s) have been altered;
or
a) a heavy chain variable (VH) region comprising or consisting of SEQ. ID. NO: 14, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: 15, or a variant thereof, wherein in said variant up to 5 amino acids have been altered, for example wherein 4, 3, 2, or 1 amino acid(s) have been altered;
or
a) a heavy chain variable (VH) region comprising or consisting of SEQ. ID. NO: 16, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: 17, or a variant thereof, wherein in said variant up to 5 amino acids have been altered , for example wherein 4, 3, 2, or 1 amino acid(s) have been altered;
or
a) a heavy chain variable (VH) region comprising or consisting of SEQ. ID. NO: 18, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: 19, or a variant thereof, wherein in said variant up to 5 amino acids have been altered , for example wherein 4, 3, 2, or 1 amino acid(s) have been altered;
or
a) a heavy chain variable (VH) region comprising or consisting of SEQ. ID. NO: 20, or a variant thereof, wherein in said variant up to 5 amino acids have been altered , for example wherein 4, 3, 2, or 1 amino acid(s) have been altered ; and
b) a light chain variable (VL) region comprising or consisting of SEQ. ID. NO: : 21, or a variant thereof, wherein in said variant up to 5 amino acids have been altered , for example wherein 4, 3, 2, or 1 amino acid(s) have been altered;
or
a) a heavy chain variable (VH) region comprising or consisting of SEQ. ID. NO: 24 ; and
b) a light chain variable (VL) region comprising or consisting of SEQ. ID. NO: 25 .

8. The antibody according to any one of the preceding claims, wherein the antibody is capable of binding to an epitope contained between residues corresponding to amino acids 105 to 117 of the myotoxin II protein sequence as defined in SEQ. ID. No 1 or variant(s) thereof having at least 70% identity, such as at least 75% identity, for example at least 80% identity, such as at least 85% identity, for example at least 90% identity, such as at least 95% identity thereto.

9. A pharmaceutical composition comprising an antibody as defined in any one of the preceding claims, and a pharmaceutically acceptable diluent, carrier, and/or excipient, optionally further comprising one or more additional antigen-binding protein(s) capable of binding to, blocking, or neutralizing one or more snake venom antigen(s).

10. A kit-of-parts comprising
a) an antibody according to anyone of claims 1 to 8; and
b) another agent suitable for the treatment of snake envenomation, preferably wherein said other agent used for the treatment of snake envenomation is one or more antigen-binding protein(s) binding to, blocking, or neutralizing one or more snake venom antigen(s) selected from the group consisting of: IgG, IgM, scFv, F(ab)₂, Fab, V_{H}H antibody(ies), and fragments thereof, even more preferably wherein the one or more antigen-binding protein(s) capable of binding to, blocking, or neutralizing one or more snake venom antigen(s) is (are) selected from the group consisting of: Equine immunoglobulin(s) against snake venom, such as Equine IgG(s) or Equine Fab or Equine F(ab')₂ fragment(s) against snake venom, Ovine immunoglobulin(s) against snake venom, such as Ovine IgG(s) or Ovine Fab or Ovine F(ab')2 fragment(s) against snake venom, Rabbit immunoglobulin(s) against snake venom, such as Rabbit IgG(s) or Rabbit Fab or Rabbits F(ab')₂ fragment(s) against snake venom, and Camelids immunoglobulin(s) against snake venom, such as Camelids IgG(s) or Camelids Fab or Camelids F(ab')₂ fragment(s) against snake venom.

11. The composition or kit-of-parts according to any one of claims 9 to 10, wherein said other agent used for the treatment of snake envenomation is one or more small molecule(s) with inhibitory effect against snake toxins, preferably wherein the one or more small molecule(s) with inhibitory effect against snake toxins is (are) selected from the group consisting of: polyanions, polyphenolic compounds, and metalloproteinase inhibitors, even more preferably wherein the one or more small molecule(s) with inhibitory effect against snake toxins is (are) selected from the list consisting of: Suramin, Heparin, Wedelolcatone, Coumestrol, Glycyrrhizin, Fucoidan, Apigenin analogue, AG-3340, Aristolochic acid, Rosmarinic acid, Imidazopyridine, BAPTA, TPEN, EDTA, DMPS, dimercaprol, BAY-129566, Marimastat, CGS-27023A, Batimastat, Nafamostat, MV 8612 from Mandevilla velutina, MV 8608 from Mandevilla velutina, Koninginin, 4-nerolidylcatechol, Anisodamine, 12-methoxy-4-methylvoachalotine (MMV), Quinonoid xanthene ehretianone, Benzoylsalireposide, Salireposide, Chlorogenic acid, Caffeic acid, 2-hydroxy-4-methoxy benzoic acid and, Varespladib.

12. An antibody according to any one of claims 1 to 8, a pharmaceutical composition according to any one of claims 9 and 11 or a kit-of-parts according to any one of claims 10 to 11 for use in a method of treatment of snake envenomation in a subject.

13. A kit for detection and/or quantification of snake venom in a sample comprising:
- one or more antibodies, such as two antibodies, wherein at least one of said antibodies is according to any one of claims 1 to 8; and
- means for detection of a complex comprising said one or more antibodies bound to snake venom.

14. Use of an antibody according to any one of claims 1 to 8 in an *in vitro* method for detection and/or diagnosis of snake envenomation in a subject.

15. A method for detecting snake venom in a sample, said method comprising:
- providing a sample for analysis;
- contacting the sample with one or more antibodies as defined in any one of claims 1 to 8 to said sample; and
- detecting a complex comprising said one or more antibodies bound to a snake antigen.
